Europäisches Patentamt

**European Patent Office**

Office européen des brevets

(11) Publication number: **0 164 876**
**B1**

(12) # EUROPEAN PATENT SPECIFICATION

(45) Date of publication of patent specification: **23.03.88**

(51) Int. Cl.⁴: **C 12 Q 1/68**

(21) Application number: **85303176.3**

(22) Date of filing: **03.05.85**

(54) Displacement polynucleotide assay employing recombination protein and diagnostic kit.

(30) Priority: **07.05.84 US 607885**
**20.12.84 US 684305**

(43) Date of publication of application:
**18.12.85 Bulletin 85/51**

(45) Publication of the grant of the patent:
**23.03.88 Bulletin 88/12**

(84) Designated Contracting States:
**AT BE CH DE FR GB IT LI NL SE**

(56) References cited:
**EP-A-0 070 685**
**EP-A-0 124 221**
**EP-A-0 130 515**
**US-A-4 302 204**
**US-A-4 358 535**
**US-A-4 395 486**

**SCIENTIFIC AMERICAN, vol. 241, no. 2, August
1979, USA; R. DEVORET "Bacterial Tests for
Potential Carcinogens", pages 28-37**

(73) Proprietor: **ALLIED CORPORATION**
**Columbia Road and Park Avenue P.O. Box 2245R
(Law Dept.)**
**Morristown New Jersey 07960 (US)**

(73) Proprietor: **GENETICS INSTITUTE, INC.**
**87 Cambridgepark Drive**
**Cambridge Massachusetts 02140 (US)**

(72) Inventor: **Collins, Mary**
**27 Euclid Avenue**
**Natick Massachusetts 01760 (US)**
Inventor: **Fritsch, Edward Francis**
**115 North Branch Road**
**Concord Massachusetts 01742 (US)**
Inventor: **Williams, Jon Ira**
**123 North Mountain Avenue**
**Montclair new Jersey 07042 (US)**
Inventor: **Brewen, Joseph Grant**
**17 Concord Lane**
**Convent Station New Jersey 07961 (US)**
Inventor: **Diamond, Steven Elliot**
**30 Park Lane**
**Springfield New Jersey 07081 (US)**
Inventor: **Ellwood, Marian Sue**
**16 Russell Place**
**Summit New Jersey 07901 (US)**

**0 164 876**

74 Representative: **Myerscough, Philip Boyd et al**
**J.A.Kemp & Co. 14, South Square Gray's Inn**
**London, WC1R 5EU (GB)**

**Description**

Background of the invention

The present invention relates to a diagnostic assay method and kit for detecting the presence of a target nucleotide sequence (either DNA or RNA) in a biological sample.

Conventional methods for detecting the presence of a particular polynucleotide in a biological sample typically involve immobilization of nucleic acid of the sample on a surface as the initial step. Once the sample is immobilized, a probe polynucleotide strand, usually tagged with a detectable label such as radioactive phosphorus atoms, is incubated with the immobilized sample so as to bind to the immobilized sample by purine/pyrimidine base sequence-specific complementary base pairing when the immobilized sample contains the target nucleotide sequence. After washing off the labeled probe which has not so hybridized, the presence or absence of label on the support is then determined. Techniques for this determination include exposure of a photographic film, liquid scintillation counting, and fluorescence microscopy. See U.S. Patent 4,358,535 to *Falkow* et al. (1982).

Ward and coworkers (see EP—A—63,879 (1982)) have described a variation of this technique in which, rather than tagging the probe directly with a detectable label, the probe is tagged with a nonisotopic substituent such as biotin on certain nucleotides. In such case, after the unhybridized probe is washed off, the support is contacted with a reagent such as avidin linked to an enzyme. The avidin-enzyme complex binds selectively to biotin because of the high avidin-biotin binding affinity, so as to affix enzyme selectively where the target nucleotide sequence has been immobilized on the support. Thereafter, a substrate for the enzyme is added and products of the enzymatic reaction are detected, yielding an amplified signal functionally dependent upon the initial concentration of target nucleotide sequence on the substrate. See also EP—A—97,373 of ENZO BIOCHEM, INC (January 4, 1984).

A variation in the above nonisotopic system has also been described in another European patent application of Standard Oil of Illinois (EP—A—70,687 (1983)) in which, in one form (see pages 8—10 thereof), two nucleic acid probes specific for the target nucleotide sequence are employed. The first probe, which can hybridize to a first portion of the target nucleotide sequence, is affixed to a solid support such that, upon incubation of the solid support with a sample of the biological material, target nucleotide sequences in the sample will bind to the support selectively via this first immobilized probe. Thereafter or concurrently, the second probe, which can hybridize selectively to a second and distinct portion of the target nucleotide sequence, is exposed to the support. Again, if the target nucleotide sequence is present in the biological sample, the second probe will bind selectively to that nucleotide sequence; and a combination structure (or sandwich) will be created linking the second probe to the support via the first probe and the target nucleotide sequence. The published patent application discloses labeling this second probe with a moiety directly or indirectly generating or absorbing specific wavelengths of light (e.g., a fluorescent label, a phosphorescent label or a chemiluminescent label). By separating the support from unbound solution constituents at each stage, the presence of label in the phase with support after the third separation will be a function of the presence and concentration of the target nucleotide sequence in the sample. See also WO 83/01459 of Orion-Yhtma Oy (April 29, 1983).

A different diagnostic method for a specific target nucleotide involving digestion of double-stranded sample nucleic acid in solution with a restriction enzyme, followed by detection of specifically sized fragments on filter paper, is disclosed in U.S. Patent 4,395,486 to Wilson et al. In that disclosure, the presence of the single base substitution causative of sickle cell anemia abolishes a specific site for restriction enzyme cleavage, and thereafter two specifically sized small fragments which are usually detected are then detected in reduced amounts (for sickle cell trait) or are absent (for sickle cell anemia).

While the above procedures will detect the presence of nucleotide sequences in biological samples in many cases, they each have the disadvantage of either multiple steps or steps with necessarily long incubation periods that make them impracticable for easy use in a clinical laboratory. Furthermore, many of these processes suffer from a limited selectivity or sensitivity with regard to interfering polynucleotide sequences or reliable detection of low levels of target nucleotide sequence against the background signal. In particular, nonspecific binding of the labeled probe to a support material represents a source of substantial background signal in each process.

Apart from the analysis of biological samples for target nucleotide sequences, various aspects of the physical chemistry of hybridization (formation of double-stranded helices between complementary polynucleotide sequences) have been studied. These studies have included examination of the phenomena of strand migration and displacement in nucleic acid, both *in vivo* and *in vitro*. By referring to such studies, however, we do not admit that the phenomena of strand migration and displacement have any obvious applicability to diagnosis and detection. C. Green and C. Tibbetts, *Nucleic Acids Research* vol. 9, No. 8, pp. 1905-18 (1981), have described the formation of a complex (hybrid) of a 6.1 kb (6100 base long) single-stranded DNA polynucleotide hybridized near its middle (the interval 1.7—3.3 kb) by an end-labeled complementary DNA polynucleotide of 1.6 kb length. Addition to this complex, in solution, of the 6.1 kb complementary strand caused rapid displacement of the labeled polynucleotide (see Fig. 2 on page 1910 of this reference), monitored by taking aliquots of the reaction mixture, separating them by gel chromatography and analyzing them by autoradiography. The displaced 1.6 kb polynucleotide increased steadily from under 10% to over 90% of the radioactive signal in a period of more than 85 minutes (depending upon concentration) with the 1.6/6.1 kb hybrid accounting for the bulk of the remaining radioactivity. The presumed partially displaced intermediate, which would have a total mass equivalent to 13.8 kb of DNA (both long strands and a partially displaced short strand) was apparently not detected. The authors concluded that the initial hybridization of the two 6.1 kb polynucleotides, forming a branched species, was the rate-limiting step; and that displacement along the 1.6 kb paired region of a labeled polynucleotide was very rapid, consistent with a calculated average lifetime of the branched (13.8 kb mass equivalent) species of 0.8 minutes. They indicate the possibility of both single-branched or doubly nucleated (D-looped) intermediate species (illustrated on page 1912 of the reference). In order to better study the phenomenon of branch migration, they attempted to slow the displacement process, by using drugs which might retard the migration phenomenon and/or by using complexes with more than 1.6 kb of hybrid base pairing (see pages 1913—1914 of the reference). It should be noted that the 1.6/6.1 kb species was challenged by Green *et al* only with the 6.1 kb complement, purified away from any non-specific strands and in the absence of any additional reaction constituents other than conventional media for nucleic acid hybridization.

The role of the protein produced at the rec A genetic locus of the bacterium *E. coli* (sometimes called and hereafter referred to an *E. coli* rec A protein) in the generalized recombination of DNA has been studied genetically and biochemically as a separate topic in several research laboratories. The action of this protein appears to involve an enzymatic role in promoting hybridization between homologous segments of different DNA strands. See C. M. Radding, *Ann. Rev. Genet.,* vol. 16, pp. 405—437 (1982). Such activity of the *E. coli* rec A protein has been exploited in *in vitro* hybridization in various contexts: (A) between complementary single-stranded DNA molecules (G. M. Weinstock et al. *Proc. Nat. Acad. Sci. U.S.A.,* vol. 76, pp. 126—130 (1979); Keener et al., *Nucleic Acids Research* vol. 12, pp. 6127—6139 (1984) (this reference, using circular single stranded molecules, may not constitute prior art with regard to the present invention); (B) between single stranded DNA and homologous duplex DNA (K. McEntee et al., *Proc. Nat. Acad. Sci. U.S.A.,* vol. 76, pp. 2615—2619 (1979) and T. Shibata et al., *Proc. Nat. Acad. Sci. U.S.A.,* vol. 76, pp. 1638—1642 (1979)); (C) between two duplex DNA molecules (E. Cassuto et al., *Proc. Nat. Acad. Sci.,* vol. 77, pp. 3962—3966 (1980) and R. P. Cunningham, *Cell,* vol. 20, pp. 223—235 (1980) and (D) in nucleic acid branch migration (M. M. Cox et al., *Proc. Nat. Acad. Sci. U.S.A.,* vol. 78, pp. 3433—3437 (1981)). Additional studies of *E. coli* rec A protein and its structural and enzymatic activities are contained in the following (and in references cited therein): J. Flory et al., *Cell,* vol. 28, pp. 747—756 (1982); S. Gottesman, *Cell,* vol. 23, pp. 1—2 (1981); K. McEntee et al., *J. Biol. Chem.,* vol. 256, pp. 8835—8844 (1981); K. McEntee et al., *Progress in Nucleic Acid Research and Molecular Biology: Multiprotein Interactions,* vol. 26, pp. 265—279 (W. E. Cohen, ed., Academic Press 1981); T. Ogawa et al., *Cold Spring Harbor Symp. Quant. Biol.,* vol. 43, pp. 909—915 (1978); T. Shibata et al., *J. Biol. Chem.,* vol. 256, pp. 7557—7564 (1981); G. M. Weinstock et al., *J. Biol. Chem.,* vol. 256, pp. 8829—8834 (1981). While, in general, proteins from other organisms having similar structure and activity to the rec A protein of E. coli have not been as well characterized, it is clear that similar proteins, with similar biological roles and activities, are present in other bacterial strains such as *Proteus mirabilis* (G. Eitner et al., *Mol. Gen. Genet.,* vol. 185, pp. 481—486 (1982)); *Haemophilus influenzae* (J. Kooistra et al., *J. Bacteriol.,* vol. 127, pp. 327—333 (1976)); *Salmonella typhimurium* (D. G. MacPhee, *J. Bacteriol.,* vol. 104, pp. 345—350 (1970)); *Streptococcus faecalis* (Y. Yagi et al., J. Bacteriol., vol. 143, pp. 966—970 (1980)); and *Bacillus subtilis* (W. M. deVos et al., *Mol. Gen. Genet.,* vol. 190, pp. 56—64 (1983). It is further expected that proteins of such activity will be found in many eucaryotic organisms; examples are already genetically defined for yeast (S. Prakash et al., *Genetics,* vol. 94, pp. 31—50 (1980); J. C. Game et al., *Genetics,* vol. 94, pp. 51—68 (1980)) and Drosophila (B. S. Baker et al., *Proc. Nat. Acad. Sci. U.S.A.,* vol. 73, pp. 4140—4144 (1976)), whose gene products are or influence proteins of similar activity.


Brief description of the invention

The present invention is based upon the displacement of a labeled polynucleotide from a probe polynucleotide by any target nucleotide sequences of a sample, wherein the displacement is enhanced (especially at lower temperatures and other more nearly physiological conditions) by the presence of: (1) a recombination protein having hybridization-enhancing activity (such as *E. Coli* rec A protein) and (2) cofactors necessary for the activity of such recombination protein, enabling direct or indirect measurement of label found in or on the displaced labeled polynucleotide (or in some cases the labeled polynucleotide not displaced). This label serves as a reliable, quantitative measurement functionally related to the presence and concentration of target nucleotide sequence in a sample. Accordingly, the present invention provides a method for determining the presence of a predetermined target nucleotide sequence (DNA or RNA) in the nucleic acid of a biological sample which comprises the steps:

(a) providing a reagent complex of (i) a probe polynucleotide which is capable of base pair binding via hydrogen bonds of purine/pyrimidine base pairs to the target nucleotide sequence, and (ii) a labeled poly-nucleotide which is bound by base pair binding via hydrogen bonds of purine/pyrimidine base pairs to the probe polynucleotide in a region of the probe polynucleotide at least partially coextensive with the region in which the probe polynucleotide is capable of binding to the target nucleotide sequence;

(b) contacting the reagent complex with a sample in the presence of (1) an effective amount of a recombination protein having hybridization-enhancing activity and (2) cofactors necessary for its activity

under conditions in which the target nucleotide sequence, if present, binds to the probe polynucleotide and displaces labeled polynucleotide from the reagent complex; and

(c) determining the presence (which can include determining the amount) either of labeled polynucleotide displaced from the reagent complex or of the labeled polynucleotide remaining in the reagent complex.

The present invention also provides a diagnostic kit for determining the presence of a target nucleotide sequence in the nucleic acid of a biological sample comprising:

(a) the reagent complex of:

(i) a probe polynucleotide which is capable of binding via hydrogen bonds of purine/pyrimidine base pairs to the target nucleotide sequence, and

(ii) a labeled polynucleotide which is bound via hydrogen bonds of purine/pyrimidine base pairs to the probe polynucleotide in a region of the probe polynucleotide at least partially coextensive with the region in which the probe polynucleotide is capable of binding to the target nucleotide sequence; the base pairing between the target nucleotide sequence and the probe polynucleotide being of sufficient cumulative binding strength (as defined below) for the target nucleotide sequence, if present in a sample with which the reagent is contacted, to be able to displace labeled polunucleotide from the reagent complex;

(b) a recombination protein having hybridization-enhancing activity; and

(c) cofactors necessary for the activity of the recombination protein.

Brief description of the figures

Figure 1A is a schematic view of one embodiment of the reagent complex of the present reagent useful in the present process;

Figure 1B is a view similar to Figure 1A in which the reagent complex is partially hybridized with a target nucleotide sequence G of sample nucleic acid;

Figure 1C is a view similar to Figure 1B, in which the target nucleotide sequence of the sample nucleic acid has begun to displace labeled polynucleotide from the reagent complex;

Figure 1D is an enlarged view similar to Figure 1C, in which the helical structure of double-stranded portions is schematically shown;

Figure 1E is a view similar to Figure 1C in which the labeled polynucleotide has been fully displaced from the reagent complex;

Figure 1F is a view similar to Figure 1B of a second embodiment of the present invention in which the immobilized polynucleotide has sequences complementary to and based paired with the sample nucleic acid on both sides of the region where the labeled polynucleotide is bound;

Figure 1G is a view similar to Figure 1A of a reagent complex according to a modification of the first embodiment;

Figure 2 is a view similar to Figure 1A of a reagent complex according to a third embodiment of the present invention, with a sample polynucleotide having the target nucleotide sequence shown prior to hybridization to the reagent complex;

Figure 3A is a view similar to Figure 1A of a reagent complex according to a fourth embodiment of the present invention;

Figure 3B is a view similar to Figure 1C of the fourth embodiment of Figure 3A;

Figure 3C is a view similar to Figure 1E of the fourth embodiment of Figure 3A;

Figure 3D is a view similar to Figure 3A of modified form of the reagent complex of the fourth embodiment;

Figure 4 is a view similar to Figure 1A of a reagent complex according to a fifth embodiment of the present invention;

Figure 5 is a view similar to Figure 1A of a reagent complex according to a sixth embodiment of the present invention; and

Figure 6 is a view similar to Figure 1A of a reagent complex according to a seventh embodiment of the present invention.

Detailed description of the invention

In this application the following terms are used based on their generally accepted meanings in the field of molecular biology:

Polynucleotide or Polynucleotide Strand refers to a linear polymeric structure of pentose sugars (generally ribose or deoxyribose) linked to each other by 3′,5′-phosphodiester linkages, and linked by carbon-nitrogen bonds at the 1-carbon of the sugar to pendant purine or pyrimidine bases such as, but not limited to, uracil (linked naturally to ribose only as rU), thymine (linked naturally to deoxyribose only as dT), cytosine (dC or rC), adenine (dA or rA) and guanine (dG or rG). Polynucleotides thus include strands of deoxyribonucleic acid (DNA) and strands of ribonucleic acid (RNA).

The ends of such Polynucleotide Strands are referred to as the Five Prime (5′) end, where the 5-carbon of the pentose is not linked to another pentose (but may bear hydroxyl, monophosphate or other natural or synthetic moieties), and the Three Prime (3′), end, where the 3-carbon of the pentose is not linked to another pentose (but may similarly bear hydroxyl, monophosphate or other natural or synthetic moieties).

Complementary Base Pairing or Purine/Pyrimidine Base Pairing refers to the hydrogen bonding

5

# 0 164 876

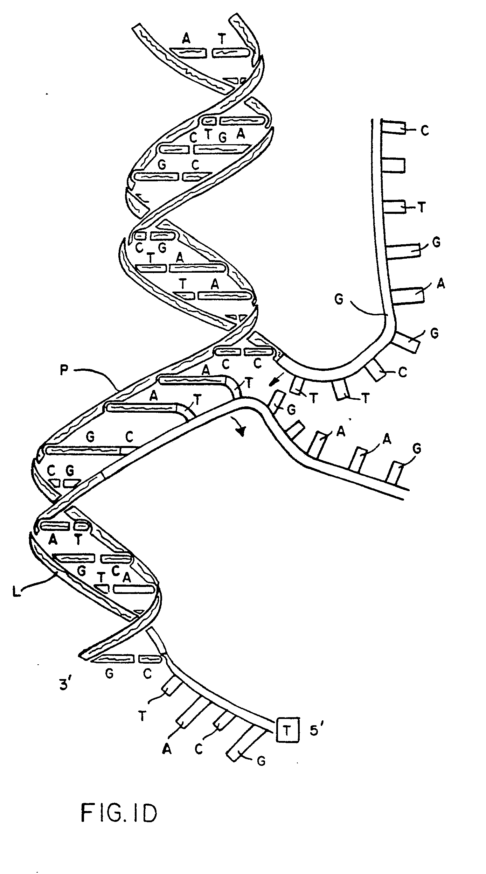

between opposite bases pendant on two antiparallel Polynucleotide Strands, which is most energetically favorable for natural DNA when dG is opposite dC and dA is opposite dT. Bases other than the five naturally-prevalent ones also have preferential pairing: for example, 5-methylcytosine binds preferentially to guanine. For illustrative purposes, this pairing is shown in many of the Figures by parallel straight lines with opposite strands directed in antiparallel directions (in the 5' to 3' sense). It should be appreciated, however, that the actual geometry of double-stranded segments will normally be helical (the well-known double helix) of various pitches, as schematically illustrated in Figure 1D.

Hybridization is used herein to refer to admixing two Polynucleotides under conditions conducive to the formation of double-stranded structures, with Complementary Base Pairing causing such double stranded structures to form where complementary sequences or nearly complementary sequences are present.

The basic components of the method of the invention are a probe polynucleotide (sometimes called herein the probe), a labeled polynucleotide (sometimes called herein tagged polynucleotide or release tag), a recombination protein (or a combination of such recombination proteins), cofactors necessary for the hybridization promoting activity of the recombination protein, and the biological sample containing nucleic acid, a portion of which is sometimes called herein the sample target polynucleotide, target polynucleotide or target nucleotide sequence. A sample may or may not contain a target nucleotide sequence. In some cases a support is also provided, either to which the reagent complex is immobilized via the probe (such that the probe is sometimes called an immobilized probe or immobilized probe polynucleotide), or in other cases as a part of the separation step that may follow displacement as a part of the determination or detecting step. In practicing the process, additional reagents or equipment are frequently required for readout; the term readout refers to the direct or indirect detection of labeled polynucleotide in one or more phases of (usually separated) reaction materials, and especially in a liquid phase by virtue of displacement from the reagent complex and separation of displaced labeled polynucleotide in solution from probe polynucleotides and reagent complexes.

In the practice of the present invention, the probe polynucleotide can be a linear or circular polynucleotide capable of binding specifically through complementary base pairing in at least one region of its purine/pyrimidine base sequence to specific target nucleotide sequences of a sample. This binding may be between DNA and RNA, between DNA and DNA or between RNA and RNA. Accordingly, the probe may either be DNA or RNA. To obtain maximum benefit of the recombination protein in the process of the present invention, it is presently preferred that the sample and probe each be DNA. As discussed more fully below, it is generally only a specific region of the probe which binds selectively to the target nucleotide sequence. Other regions of the probe may be of various naturally occurring or synthesized sequences which do not participate in the hybridization reaction with the target nucleotide sequence, but which may play an important role in the present invention, e.g., by serving as a site for attachment to a support or by providing some degree of separation between the support and the region to which the target nucleotide sequence binds, if desired.

Referring to the region of the probe to which the target nucleotide will specifically bind, called herein the target binding region (TBR in the Figures), the binding may be (and preferably is) perfect, in the sense that each nucleotide in the sequence finds its correct complementary binding partner (e.g., dA to dT) in the target nucleotide sequence or may contain some mismatches. At least one portion of the target binding region of the probe is preferably single-stranded in the reagent complex, i.e., it is not complementary to labeled polynucleotide sequences; this single-stranded region is sometimes called herein the initial binding region (IBR in the Figures) because the target nucleotide sequence can bind to this region of bases without displacing any of the labeled polynucleotide. Such initial binding region of the probe can be at least fifteen bases in length, and is preferably at least fifty bases in length if the recombination protein functions by catalyzing hybridization of single strands as illustrated in the Figures. There are other instances, however, where other activities of the recombination protein may be relied on, in which case little or no single-stranded region (IBR) need be present to achieve displacement. See D. A. Soltes et al., *J. Biol. Sci.*, vol. 259, pp. 12020—12024 (1984) (one instance of completely double-stranded hybrid except for a 3' overhang of four bases on each strand). The overall target binding region includes the initial binding region and most or (preferably) all of the labeled polynucleotide binding region (LBR in the Figures and in the discussion below). The length of the overall target binding region is not independently critical, but rather can be considered as a function or sum of the preferred or more preferred lengths of the IBR and LBR portions. Base lengths of the initial binding region of the probe above five hundred are generally not required, but are not significantly disadvantageous in most cases. A suitable lower limit on the length of this region of potential base pairing for clinical laboratory and other commercial applications is somewhat dependent upon base sequence of the target binding region of the probe polynucleotide and base composition and other physical factors described below, and especially upon the minimum length of potential pairing for significant activity of the recombination protein (See Keener et al., *Nucleic Acid Research*, vol. 12, pp. 6127—6139 (1984)), the conditions for intended hybridization, mode of attachment, if any, of the probe to a support, kinetics of hybridization and the readout system employed.

The solid phase to or on which the probe can be immobilized in certain embodiments may be of almost any conventional type, including especially polymeric materials, ceramic materials, walls of a test tube or other container, paper, nitrocellulose or glass. In some forms of the invention, the solid phase consists of

6

natural, modified natural or synthetic particles or beads made of materials such as protein, fixed cells or viruses, various polymers such as polystyrene, latex or glass.

The means of attachment of the probe to the solid support may be simple adsorption, but is preferably some form of specific covalent linkage, ionic bonding, hydrophobic interaction or hydrogen bonding. In the case of covalent linkage, the binding may be direct as by reaction between chemical moieties on the surface of the support (for example, amine or carboxyl moieties) and moieties on the polynucleotide, and especially hydroxyl or phosphate moieties on the end sugar rings of the polynucleotide. Linking agents which are specific to the free secondary hydroxyl normally present at the 3' end include phosphites, succinic anhydride and phthalamide. Linking agents which are specific to the phosphate normally present on the sugar at the 5' end (at least for most naturally occurring polynucleotides or products of most cleavage reactions) include carbodimides such as 1-ethyl-3,3'-dimethylaminopropylcarbodiimide, with or without imidazole or 1-methylimidazole. See B.C.F. Chu et al., *Nucleic Acids Research*, vol. 11, No. 8, pp. 6513-29 (1983). Such linkages which are specific to an end or other small portion of the probe, if remote from the target binding region, may permit greater degrees of freedom during the hybridization reaction compared to adsorption or other similar physical or non-specific chemical means of attachment. With such greater degrees of freedom, the minimum length of the target binding region or minimum time for the hybridization may be lowered.

Non-specific covalent linkages include linkages between the substrate and free bases along the chain via moieties such as m-aminobenzyloxy methyl (ABM), m-diazobenzyloxy methyl (DBM) or o-aminophenylthioether (APT). See H. Bunemann et al., *Nucleic Acids Research*, vol. 10, No. 22, pp. 7163—7196 (1982) (two articles). Other exemplary non-specific linking chemistry is described in U.S. Patent 4,286,964 to B. Seed (1981).

In addition to direct covalent linkage, the probe polynucleotide may be indirectly linked to the support by a linking or spacer molecule. Examples of indirect covalent linking reagents include spacer reagents which can react by carbodiimide chemistries both with functional groups (for example, esters) on the support and with the phosphate normally present on the 5' terminal sugar of the polynucleotide. Such spacer molecules include the aliphatic diamines used in the above-cited Chu et al. article which, once attached to the terminal phosphate, can then be linked to active groups on the support such as N-hydroxysuccinimide esters. Other spacer molecules include hydroxyalkanoic acids.

Still other spacer molecules can contain a functional moiety such as phenyl ketone which will react directly with a support having hydrazine moieties, forming a resultant hydrazone.

The probe further may be noncovalently linked to the support by interaction of some portion of the probe with affinity reagents that are adsorbed or covalently bound to the support. Examples include (1) immobilization on the support of a short single-stranded polynucleotide which can hybridize to some portion of the probe polynucleotide not overlapping with the region in the probe which is capable of binding to the target nucleotide sequence and (2) binding of a chemically modified probe polynucleotide carrying one or more avidin or biotin moieties to a support having biotin or avidin moieties, respectively, adsorbed or covalently bound to the support. The latter method is based on the high affinity ($K_{diss}$ approximately $10^{-15}$ M) of the small molecule biotin for the protein avidin (or streptavidin).

While the present invention is not limited with regard to the spacings between the point or points of attachment of the probe to a support and the region of the probe polynucleotide which binds specifically to the target nucleotide sequence, it is preferable that this spacing be sufficiently large for the hybridization between target nucleotide sequence and target binding region of the probe polynucleotide to occur such that the target binding region of the probe has sufficient, and preferably maximal obtainable freedom of movement during hybridization.

In other embodiments of the invention, the probe polynucleotide is not immobilized on a support, but rather the entire reagent complex is in solution as the reagent is mixed with a biological sample such that hybridization will occur, if at all, in solution. In some of those solution hybridization embodiments, the probe does contain a substituent (such as an affinity reagent, e.g., biotin) so as to have the probe be immobilizable or separable if desired after hybridization, e.g., by passing the reaction mixture through a porous bed or filter with streptavidin bound to the support matrix. Such immobilization will cause only displaced labeled polynucleotides to remain in the liquid phase, for subsequent determination. Still other forms of the invention involving solution hybridization include alternative methods of separations such as size exclusion chromatography, electrophoresis (see Examples below), or other physical separation techniques. Additional forms of the invention, described more fully below in connection with readout, involve determination of displaced labeled polynucleotide without any separation from complex. Of course many of such determinations without separation apply equally to processes wherein the complex includes an immobilized probe polynucleotide.

Such probe polynucleotides can be manufactured reproducibly in a variety of ways, e.g., cloned in or as a part of a suitable plasmid or viral vector isolated form a bacterium or other suitable microorganism, as a part of the genetic material or a microbe or obtained from any other pertinent biological source. Generally, only a small region of nucleic acid that includes a probe polynucleotide sequence (a portion of which forms the target binding region) will be inserted into any such cloning vectors by recombinant techniques; the remainder, if any, of the cloned insert that is not probe polynucleotide sequence can conveniently be chosen from any polynucleotide sequence heterologous to the target nucleotide sequence.

In certain embodiments of this invention, such heterologous sequences can include sequences deliberately selected for specific properties such as the presence of a unique restriction enzyme recognition site. Under some conditions an entire gene or a sequence including an entire gene may be used as an insert, with the vector plus inserted nucleotide sequence either in circular. or linear form. In the event that the probe polynucleotide is double-stranded when manufactured, denaturation of the probe by thermal means, by adjustment of pH or by disruption of base pairing with other conventional techniques will normally follow isolation. Cleavage (especially by restriction enzymes or by site-specific chemical cleavage) will normally be used to form double-stranded segments of desired linear form and, if double-stranded circular forms are obtained, will precede denaturation. In some cases it may be preferred to purify individual strands from a double-stranded structure either to be used individually as probe polynucleotides or with one as a probe polynucleotide and the other as the precursor for the labeled polynucleotide. This purification can be done by standard techniques such as denaturing gel electrophoresis or affinity chromatography. In some instances, the probe polynucleotide is produced as a single-stranded molecule by replication using single-stranded vectors such as M13 bacteriophage. In some other instances, as described below, the labeled polynucleotide and probe polynucleotide can be manufactured as parts of the same molecule.

The labeled polynucleotide used in the present method and reagent is generally a smaller piece of either DNA or RNA than the probe polynucleotide and has two features of significance: (a) stable but reversible binding to the probe at a specific locus and (b) a label susceptible to detection, especially after displacement. These features are discussed herein separately, followed by consideration of the effect certain types of labeling have on stability and displacement.

The pairing between the labeled polynucleotide and the probe polynucleotide will generally occur over a smaller number of bases than the pairing between the target nucleotide sequence and the probe. In most cases, the bases of the probe polynucleotide to which the labeled polynucleotide specifically binds are a subset of the bases of the probe later binding to the target nucleotide sequence of the sample nucleic acid, and thus represent a portion of what is called above the target binding region of the probe.

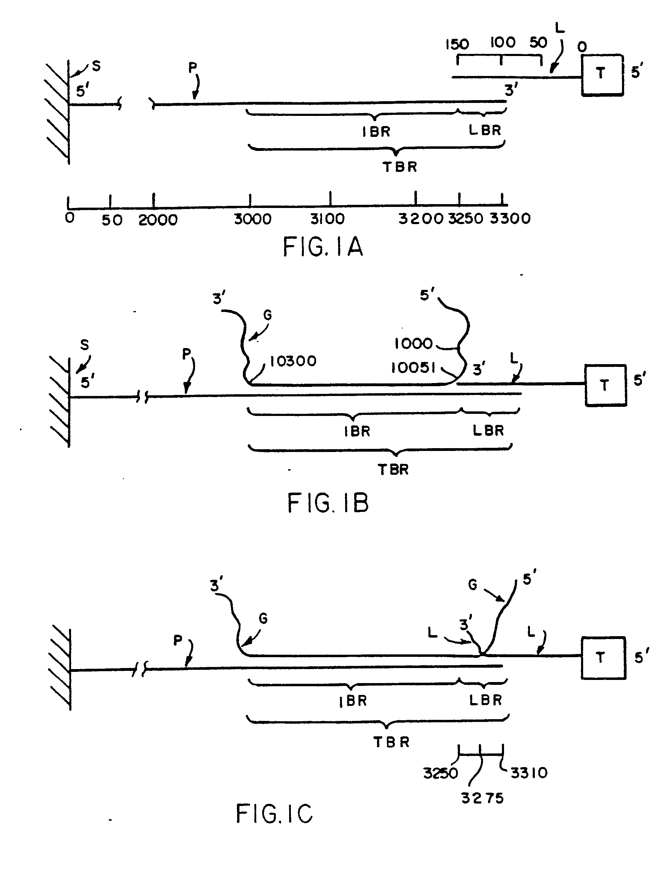

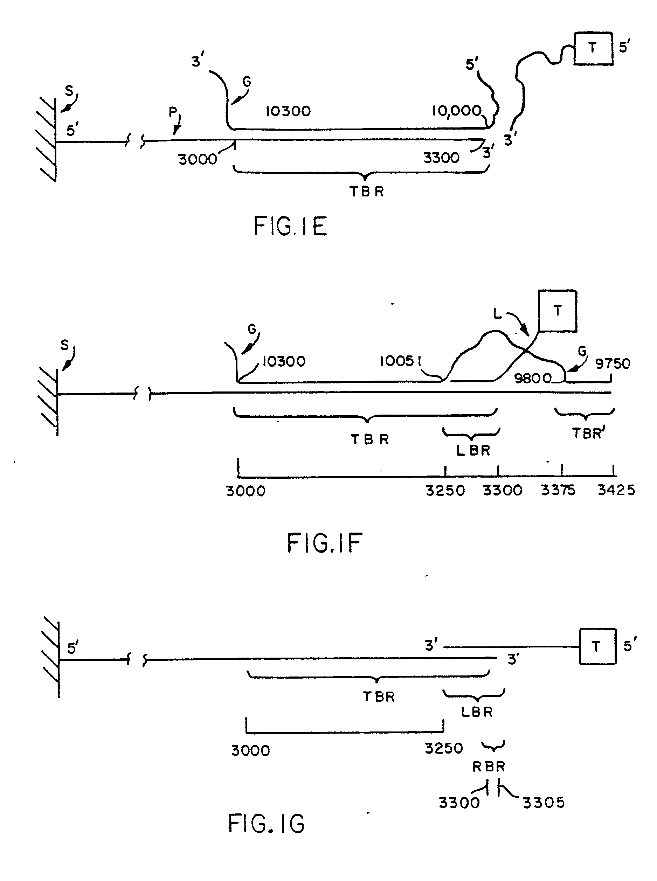

The term labeled polynucleotide binding region (LBR) is used herein to refer to that sequence of bases in the probe to which the labeled polynucleotide is bound in the complex. In preferred embodiments (as illustrated by all Figures) the labeled polynucleotide binding region is totally part of the target binding region (see especially Figure 1A); in other embodiments (e.g., see Figure 1G) only a portion (but preferably the major portion) of the labeled polynucleotide binding region is a part of the target binding region. Lengths of labeled polynucleotide binding region outside the target nucleotide binding region of the probe polynucleotide region that are greater than about 15 bases are not preferred because of the potential difficulty in disassociating the labeled polynucleotide from the probe once the only attachment between the two strands is via base pairing in this region. In some of the preferred embodiments, the region of the probe polynucleotide to which the labeled polynucleotide binds is a subset at or near one end of the larger region of the probe polynucleotide to which the target nucleotide sequence of the sample subsequently binds. In some such embodiments, if the probe is immobilized in the reagent.complex, the aforementioned one end of the larger region is at or near an end of a linear probe polynucleotide (as illustrated in Figure 1A and discussed below; note, however, that the other end of the TBR as shown in Fig. 1A may also be used as a LBR).

The present reference to a labeled polynucleotide and a probe polynucleotide as distinct entities should not be understood, however, to be a requirement that they are linked solely by complementary base-pairing or that there is necessarily only one labeled polynucleotide attached to each probe polynucleotide or that there is only one labeling moiety (tag) per labeled polynucleotide. Other forms of attachment of probe to labeled polynucleotide may also be present, but are generally not preferred. In embodiments without a separation, severing such other attachment of probe to labeled polynucleotide may or may not be required, depending upon whether or not displaced labeled polynucleotides still attached by such other means appear in the detection method as if they were totally displaced. Severing such other attachment before, during or after displacement but, in any event, before the determination step is preferred. Multiple labeled polynucleotides on a single probe strand (as illustrated in Figure 6) is a form of the invention which may have special application when greater numbers of displaced tags per displacement event are desired.

The size of the labeled polynucleotide binding region of the probe is not itself critical to the present invention. A generally preferred size for labeled polynucleotide binding region (and the corresponding base sequence of the labeled polynucleotide) is at least about 15 bases, preferably at least 20 bases, and more preferably at least about 25 bases. As with the size of the target binding region TBR, the size of the labeled polynucleotide binding region LBR may be subject to some minimum based upon limitations of the recombination protein if full advantage is to be taken of all activities of the recombination protein (cf. DasGupta et al., *Proc. Nat. Acad. Sci.*, vol. 79, pp. 762—766 (1982); Gonda et al., *Cell*, vol. 34, pp. 647—654 (1983); Bianchi et al., *Cell*, vol. 35, pp. 511—520 (1983)). A preferred range is about 20 to about 500 bases (with up to 1000 bases being also preferred), especially about 25 to about 200 bases. Labeled polynucleotides with unusually short pairing segments (giving regard to factors such as GC base content) will dissociate from the probe in a non-specific manner if the temperature of the displacement step is too high (also giving consideration to factors such as salt concentration which affect melting temperatures). See D. Freifelder et al, *Biopolymers*, vol. 7, pp. 81—93 (1969). There is no essential advantage in unusually long pairing segments (e.g., over 1000 bases). Such long pairing segments are sometimes less preferred because the overall target binding region of the probe could then become longer and require necessarily longer target sequences for successful displacement of the labeled polynucleotide. The binding portion of the labeled polynucleotide may also, if too long, no longer easily be manufactured by certain techniques available or best suited for small polynucleotides: e.g., organic chemical synthesis of the entire labeled polynucleotide. Organic chemical synthesis is generally easier at present for labeled polynucleotide binding regions of less than about 100, and especially less than about 60 nucleotides; however, improvements in such synthetic techniques could make longer sequences easy to make as well.

The minimum length of labeled polynucleotide (and also of labeled polynucleotide binding region) is related primarily to reagent complex stability. Factors other than length which affect this stability include GC content (whose effect on melting temperature is well known), internal base pair mismatches and, in the present invention, the presence of a recombination protein and its appropriate cofactors. Melting temperature (Tm) is a useful way to establish an effective minimal length for base paired sequences in reagent complexes having one or more internal base pair mismatches. As an example, a sequence of 30 bases having one internal base pair mismatch (and thus only 29 exact pairs) may perform (at least with regard to reagent complex stability) effectively like a shorter exactly matched sequence; the degree of departure from behavior as a 29 base pair sequence will depend upon the position of any base pair mismatches, the base change or changes that have been made and the concentration of recombination protein. Effective length can be expected to differ depending upon whether the mismatched pair is of the purine/purine, purine/pyrimidine or pyrimidine/pyrimidine type. Any effective length can be empirically determined, however, by control experiments in which a series of probe/labeled polynucleotide complexes are subjected to various regimes. For purposes of the present invention, such control experiments should be performed in the same solution (including cofactors and other ions such as rec A buffer) with the same amount and concentration of recombination protein (such as *E. coli* rec A protein) as would be used for contacting step (displacement reaction). By determining a melting temperature of a complex of probe with a labeled polynucleotide containing base pair mismatches, and comparing that value with melting temperatures of complexes with slightly shorter lengths of pairing (but perfect base pair matching within the period region) on the same probe polynucleotide, the effective pairing length of the labeled polynucleotide with base pair mismatches can be estimated and applied to the above preferred and more preferred ranges. The above correlations based upon melting points are intended, however, primarily as an easy estimation tool. The actual degree of preference for a given labeled polynucleotide binding region is based both on how well the labeled polynucleotide actually stays in the complex during storage of the reagent or contact with non-homologous nucleic acids under use conditions and on how well the labeled polynucleotide is displaced by nucleic acids having the appropriate target nucleotide sequence. While mismatches are permitted, they are in general not preferred and, when present, generally comprise no more than two fifths and preferably comprise no more than one tenth of the region of pairing (e.g., preferably a maximum of three mismatches and twenty-seven perfect matches in a 30 base pair long region of labeled polynucleotide/probe polynucleotide pairing). Again, the activity of the recombination protein may be affected by the degree of mismatches (c.f. DasGupta et al., Gonda et al., and Bianchi et al., cited above).

The labeled polynucleotide may contain regions of nucleotides, in addition to the pairing region, which do not specifically bind to the probe polynucleotide. Such regions may serve to link the pairing region to the detectable tag, may themselves be tagged (e.g., by radioactive labeling or by covalent attachment to an indirect marker such as avidin or biotin) or merely be present without any particular function. The labeled polynucleotide may itself be linear or circular and may be (but is preferably not) double stranded in regions other than the pairing region. The labeled polynucleotide is preferably not circular when the probe polynucleotide is circular because of expected topological constraints on hybridization of two circular nucleic acid molecules and subsequent stability of such reagent complexes.

One or more detectable tags may be generally located (using conventional techniques) at one or more of several points along the labeled polynucleotide (especially if the tag is a radionuclide or biotin or the like), only at one end or only at one specific internal location on the labeled polynucleotide (e.g., at a purine or pyrimidine base not involved in base pairing to the probe polynucleotide). Provided that there is at lesat one region of the labeled polynucleotide unpaired in the reagent complex, the tag is preferably present or concentrated on or within such unpaired region. Directly detectable tags which may be used include radionuclides (especially phosphorus-32, sulfur-35, carbon-14 or iodine-125 labeled nucleotides), fluorescent compounds (such as fluorescein or rhodamine derivatives attached to the free end of a labeled polynucleotide or to one or more of the unpaired bases of a labeled polynucleotide) or moieties directly detectable by other means (including being cleaved off) such as the moiety nitrophenol, which can be subsequently detected colorimetrically or by other means. Indirectly detectable tags include those modifications that can serve as antigenic determinants, affinity ligands, antigens or antibodies recognizable through immunochemical or other affinity reactions such as described in EPA 63,879, WO 83/02277 and EPA 97,373, referenced above and exemplified by biotinated nucleotides present in or added onto the labeled polynucleotide (such as by the enzyme terminal deoxynucleotidyl transferase which will add multiple nucleotides at the 3' end of the labeled polynucleotide in the absence of a template strand). Other indirect tags include enzymes attached to a labeled polynucleotide (especially at a free end remote from the region paired to the probe) whose presence can be determined after displacement and separation steps of the embodied method by addition of the substrate for the enzyme and quantification of either the enzymatic substrate or, preferably, the enzymatic reaction product. The use of enzymatic tags in the present invention is particularly benefitted by the neny-physiological temperature and other conditions at which the contacting (displacement) reaction is conducted. By reducing temperature compared to the preferred temperatures employed in the absence of recombination protein, problems of enzyme inactivation or denaturation are significantly avoided. Furthermore, the ion identities and concentrations during displacement can, in some such cases, more closely resemble those desired for the enzymatic reaction stage (part of the detecting step) such that fewer manipulations can be required before the detecting step. Furthermore, the tag may be an apoenzyme, co-enzyme, enzymatic modifier, enzymatic cofactor or the like, with the other necessary reagents usually added after displacement (and, in certain embodiments, after separation) along with the appropriate enzymatic substrate. Of course, if the enzymatic reaction cannot occur with all but one component present (e.g., the substrate), then these other reagents may be present in solution during the contacting (displacement) step (b) described above.

Multiple detectable tags can be added in manufacturing the labeled polynucleotide such as by using a terminal deoxynucleotidyl transferase enzyme and sufficient concentrations of all appropriate natural or modified deoxynucleotidyl triphosphates. Multiple labeled polynucleotides, e.g., one containing the enzyme (or apoenzyme) and one containing the coenzyme, can also be used. One form of attachment of an enzyme to the labeled polynucleotide is via affinity reagents, e.g., streptavidin-enzyme conjugate bound to biotin located in the polynucleotide chain. Such a binding form can be used in various embodiments, for example wherein the complex is prepared by hybridizing a biotin-labeled polynucleotide to the probe and then binding a streptavidin-enzyme conjugate to the biotin prior to the contacting (displacement) step (b) described above. Furthermore, a moiety interacting with the detectable tag in the complex may be present on the probe.

Most forms of detectable tags, especially if remote from the pairing region of the labeled polynucleotide to the probe, will have little or no effect on the strength of base pairing between the labeled polynucleotide and the probe polynucleotide, as evidenced (for testing purposes) by little or no diminution of the reagent complex melting temperature and, more importantly, by negligible effects on the hybridization reaction between any target nucleotide sequence and the probe polynucleotide. Some forms of labeling, such as covalently bound biotin on nucleotides of the labeled polynucleotide in the base pairing region and such as a large enzyme molecule or fluorescent moiety linked to nucleotides in or near the base pairing region, may have an appreciable effect on reagent complex stability. Such effect generally will be to destabilize the labeled polynucleotide/probe polynucleotide binding (and thus lower its melting temperature). That effect may be somewhat beneficial in speeding up displacement, but can cause increases in non-specific dissociation or "fall-off" of the labeled polynucleotide. Such non-specific "fall-off" can usually be reduced, however, by lowering the temperature during the displacement step such as into the physiological range, increasing the length of the labeled polynucleotide binding region, or other such modification of the physicochemical properties of the system.

Forming a reagent complex between an immobilized probe polynucleotide and a labeled polynucleotide (such complex being provided in the present process and being in the present reagent) may involve attachment of the probe to a support (as described above) either before or after hybridization of the labeled polynucleotide to the probe polynucleotide. Affinity or other chemical reagents may be used for mediating or participating in such attachment. If the immobilization is completed after hybridization of the labeled polynucleotide to the probe polynucleotide, then the linking moiety or a part thereof can be already attached to the probe. Generally, the formation of such an immobilized complex will be followed by washing off unbound labeled nucleotide, and the conditions of such washing may be designed to also remove labeled polynucleotides that are only slightly bound (e.g. through less than about fifteen complementary bases elsewhere on the probe, instead of through the larger number of complementing bases at the desired binding site) or are absorbed to the support. Probe polynucleotides and complexes of probe polynucleotide with labeled polynucleotide that are only marginally attached to the support may also be removed during this washing step. The washing should preferably be under sufficient conditions and for a sufficient time to substantially eliminate the non-specific background signal due to labeled polynucleotides (with or without probe polynucleotide) separating from the support independently of specific displacement during the displacement step of the present method.

In the manufacture of reagent complexes which are in solution when used, it is frequently also desirable to separate labeled polynucleotides which have not bound to probe polynucleotides from product reagent complexes (in some instances unbound probe polynucleotides may also be removed). In some cases, an excess of labeled polynucleotide is preferably used to minimize the presence of unhybridized probe polynucleotides. Such separation may be by size alone (e.g., by size exclusion chromatography) if, as is frequently the case, the labeled polynucleotide is such smaller than either probe polynucleotides or reagent complexes. Such separation may also be based upon the double stranded nature of at least one portion of the reagent complex (at the labeled polynucleotide binding region of the probe) where such a double stranded region is not likely to be present in either the labeled polynucleotide or probe polynucleotide, both of which would be expected to, be in single-stranded form, except for very small internal binding regions. This property renders reagent complexes separable from unbound labeled

**0 164 876**

polynucleotides by, e.g., affinity chromatography using double-strand specific anti-nucleic acid antibodies or hydroxylapatite chromatography.

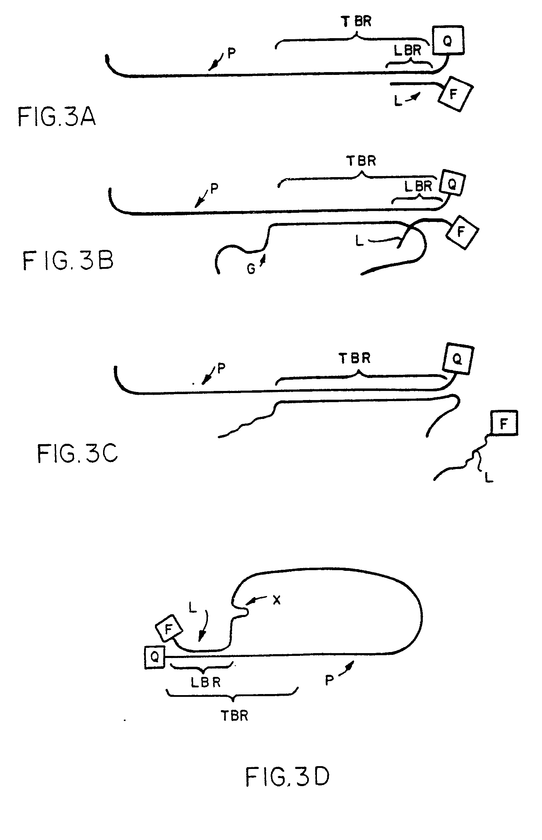

In some instances, the labeled polynucleotide and probe polynucleotide can be part of the same polynucleotide chain. For example, a linear single-stranded DNA molecule can be constructed from an M13 bacteriophage which contains a cloned DNA insert with inverted repeats. These inverted repeats, which are capable of forming a double-stranded region due to their complementarity, would include the labeled polynucleotide and the labeled polynucleotide binding region. Sequences located between or adjacent to these repeats would include the initial binding region, located adjacent to the labeled polynucleotide binding region. Unique restriction enzyme cleavage sites (e.g., the M13mp7 polylinker or modification thereof), located outside of the inverted repeats and the initial binding region, could be cleaved to release the cloned insert from the single-stranded M13 vector backbone (cf. G.A. Ricca et al, *Proc. Nat. Acad. Sci. U.S.A.*, vol. 79, pp. 724—728 (1982)). An additional small inverted repeat sequence, containing a restriction enzyme cleavage site (e.g., the M13mp7 polylinker) could be placed between the labeled polynucleotide repeats. Cleavage at such a site (e.g., X of Figure 3D of parent application U.S.S.N. 607,885) would leave only complementary purine/pyrimidine base pairing to hold the labeled polynucleotide attached to the now distinct probe polynucleotide and would provide free ends on the probe polynucleotide through which attachment to a solid support could be mediated if so desired. Any tag or tags may be added to the labeled polynucleotide at this point if not already present.

The recombination protein used in the method and kit in accordance with the present invention is illustrated by rec A protein from *E. coli*, described by C. M. Radding et al, *Ann, Rev. Genet.*, vol. 16, pp. 405—437 (1982) (and references cited therein). This protein is effective in promoting hybridization when sufficient amounts of *E. coli* rec A protein and necessary cofactors are present. In the case of *E. coli* rec A protein, these cofactors are $Mg^{++}$ (generally present at a concentration of at least 10mM and as much as 25mM or even more) and adenosine triphosphate (ATP) (generally present at concentrations of 0.5—10 mM). In addition, a system for the regeneration of ATP (from byproduct ADP), such as creatine phosphokinase and creatine phosphate, can be included, especially with lower ATP concentrations. Also, a weight ratio of rec A protein relative to the reagent complex of at least 1:1 can be desirable, with a weight ratio of 5:1 or greater being preferred and of 20:1 or greater being more preferred. *E. coli* rec A protein is preferably present in a ratio of at least 1 monomer of rec A protein per 2 to 3 nucleotides total single stranded DNA.

The *E. coli* rec A protein can be packaged together with the reagent complex in the method described herein since, in general, it does not promote spontaneous dissociation of the labeled polynucleotide from probe polynucleotide even at physiological temperatures. It is contemplated, however, that the *E. coli* rec A protein or a concentrate of rec A cofactors or both can also be a separately packaged reagent or reagents which are added in any sequence to the reagent complex before, during or, less preferably, after admixture with the sample. It is preferred that the *E. coli* rec A protein and ATP not be present in solution together for a long time at physiological temperatures before strand displacement is initiated because of the potential build-up of ADP due to rec A mediated ATP hydrolysis, which may inhibit rec A activity. See Example 2 and T. Shibata et al., *J. Biol. Chem.*, vol. 256, pp 7565—7572 (1981).

Other proteins such as those cited earlier from other microorganisms that have the same or similar nucleic acid strand synaptenic activity as rec A protein from *E. coli* can also be used in the practice of this invention. One such similar protein is the Beta protein of bacteriophage lambda (E. Kmiec et al., *J. Biol. Chem.*, vol. 256, pp. 12636—12639 (1981)).

One can, in general, use for the method and kit of the present invention any protein which will catalytically promote either (1) hybridization of two single strands, (2) exchange of strands between a single-stranded molecule and a double-stranded molecule, (3) exchange of strands between two double-stranded molecules; or more than one of these three reactions of nucleic acids. Such activities can be detected for any candidate protein in model hybridization or strand exchange reactions of the type described in various articles referenced above, and especially C. M. Radding et al. (1982). The general term, "recombination protein" is thus used herein to describe all catalytically similar proteins having one or more of these activities. It will be appreciated that the same or different cofactors could be necessary for different such proteins, and that some biological energy source (usually ATP) will generally be one of those necessary cofactors.

The actual contacting or displacement step with sample material potentially containing nucleic acids that include the target nucleotide sequence will normally be under conditions of temperature, ionic strength, recombination protein concentration, cofactor concentration, and time less stringent (and thus less conducive to uncoupling of the labeled polynucleotide) than the above washing step. A desirable temperature range during the contacting step is from about 15°C to about 60°C, depending upon the solution ionic strength and other additives affecting melting temperature; the most efficient temperatures will be one at which a maximum or near maximum rate of hybridization of sample nucleic acids to probe occurs. In most cases, however, for maximal activity of the recombination protein, a temperature near physiological temperature (e.g., 25—45°C) will be used. As described in a number of literature references (e.g., J. G. Wetmur and N. Davidson, *J. Mol. Biol.*, vol. 31, pp. 349—370 (1968) and C. Minson and G. Darby, *New Developments in Practical Virology*, vol. 1, pp. 185—229 (Alan Liss, Inc., N.Y., (1982)), hybridization rate is also a function of pH and sample nucleotide concentration. In particular, pH would often be selected to achieve optional beneficial activity of the recombination protein (near pH 7.5 in the case of *E. coli* rec A protein) and a buffer such as Tris-HCl can be used to establish and maintain such pH during the reaction. Furthermore, it is contemplated that, in at least some embodiments of the invention, a volume-excluding polymer which is non-ionic or anionic (such as a poly(ethylene oxide)) may be present during this step to further enhance hybridization, as described more fully in EP—A—167238.

In addition to the cofactors necessary for rec A protein's activity, it is contemplated that other cofactors such as *E. coli* single stranded binding protein may also be present. See D. A. Soltis and I. R. Lehman, *J. Biol. Chem.* vol. 258, 6073-77 (1983) for an example of how *E. coli* rec A protein and *E. coli* single stranded binding protein may interact. See also T. Shibata et al., *Proc. Nat. Acad. Sci. U.S.A.*, vol. 77, pp. 2606—2610 (1980). Applicants have undertaken limited experimentation with the combination of *E. coli* rec A protein and single stranded binding protein and observed no obvious beneficial effect for the combination. It is anticipated that beneficial effects of such single stranded binding protein (or other proteins which interact with the recombination protein) may be realized only in certain forms of the invention, involving certain separation steps (or no separation step), certain orders of addition of components to the reaction or certain geometrics of the reagent complex and target nucleotide sequence. It is significant, however, that single-stranded binding protein is also known in the literature to promote *E. coli* rec A-moderated strand hybridization or strand exchange in some situations (see K. McEntee et al. (1980); T. Shibata et al. (1980) and M. Cox et al. (1981)) but not in others (see S. Keener et al. (1984), cited above).

Proportions, amounts and concentrations of reagent complex, recombination protein and cofactors are not independently critical, but it is generally desired that the total hybridization mixture of sample nucleic acid and reagent complex be as concentrated as feasible. In most instances, probe polynucleotides bearing binding sites for a target nucleotide sequence will be expected to be present in ten-fold or more molar excess (possibly hundred-fold or more excess) of any anticipated level of target nucleotide sequence in the sample. The sample itself may include nucleic acids which preferably should be completely or partly in solution (separated from membranes and the like) and in single-stranded form for the hybridization step of the assay. The presence of the complement of the target nucleotide sequence (by virtue of denaturation of double-stranded sample DNA) could represent an interference. This interference is likely to be minor in at least the preferred forms of the invention; in hybridizations involving immobilization of the probe selectively (before or after displacement), displaced labeled polynucleotide will be and will remain in the solution phase and can be subsequently determined, whether or not such displaced labeled polynucleotide has rehybridized with complementary segments of the sample nucleic acid. In many solution hybridizations this inferference also may be minimized by kinetic effects.

In general, strand displacement reactions using *E. coli* rec A protein should require no more than three hours, generally under one hour and desirably less than thirty minutes to achieve sufficient displacement of labeled polynucleotide for detection. Conditions often can be adjusted to achieve substantial completion of strand displacement reactions within these times. Longer incubation times are not necessarily disadvantageous, however.

In some of the forms of the invention in which the complex is initially on a solid support, the liquid phase containing displaced labeled polynucleotide may be separated therefrom as a part of the determination step. If the complex is in solution, some forms of the invention involve treatments after the contacting (displacement) step to fix reagent complexes still present (and, usually unavoidably, other forms of probe including those hybridized to a target nucleotide sequence) to a solid support, followed by a similar solid/liquid separation. Such separation of the solid phase containing bound complex from liquid phase containing displaced labeled polynucleotide may be by physical means such as chromatography, filtration, centrifugation, magnetic attraction or decantation. The solid phase may include magnetic or other separable particles that are attracted or otherwise physically removable from the liquid phase. Furthermore, complete separation of the two phases is not required; an aliquot of the liquid phase following partial separation may be removed for label determination, leaving the solid phase admixed with the remainder of the liquid phase. If such a separation occurs or is utilized, determination of the presence and frequently the quantity of labeled polynucleotide present may be conducted upon either phase, but is preferably conducted upon the liquid phase. Determination of the liquid phase for the presence and quantity of displaced labeled polynucleotide typically has the advantage of generally lower background signals. Any background signal will be largely that caused by non-specific "fall-off" from a solid support as described above, by non-specific dissociation of labeled polynucleotides from reagent complexes of by imperfect preparation of reagents. Additionally, the absence of target nucleotide sequence in the sample results in the absence of signal from labeled polynucleotide inqthe liquid phase. By contrast, if detection is of label associated with the solid phase, a negative result is indicated by unreduced levels of labeled polynucleotide. Especially when a high molar excess of reagent complex over anticipated target nucleotide sequences is used, measurement of the label remaining on the solid support potentially results in reduced sensitivity for the diagnostic method of this invention.

In some embodiments of the invention, wherein no post-displacement separation occurs, determination can still be made of displaced labeled polynucleotides. Some such determination steps involve a change in the signal detectable from a tag by virtue of displacement, including those signals affected by proximity of the tag to a signal-affecting moiety elsewhere on the labeled polynucleotide or on the probe polynucleotide (see description of Figures 3A—3D).

12

**0 164 876**

In other forms, especially with immobilized probes, the interaction can be beween two types of tag moieties. One type of detectable tag can be on labeled polynucleotides hybridized to immobilized probe polynucleotides at one location on a solid support. A second type of tag may be on labeled polynucleotides which are hybridized to immobilized probe polynucleotides at a remote location on the same solid support. The second type of tag may also be otherwise directly attached to some remote location of the probe or of the same labeled polynucleotide or of the solid support. In all such cases involving two types of tags, the two different tags can interact only if at least one labeled polynucleotide (tag) is displaced. Such interaction is especially applicable to apoenzyme with coenzyme: e.g., apoglucose oxidase with flavin adenine dinucleotide (FAD).

The process and reagent of the present invention can be used for the detection and determination of a variety of target nucleotide sequences in a variety of concentrations. In particular, microorganisms including infectious agents whose nucleic acid (genomic or otherwise) can be targeted include pathogenic viruses, bacteria and fungi; e.g., cytomegalovirus or *Neisseria gonorrhea*. Exemplary genetic disorders or conditions which can be targeted include β-thalassemias, $a_1$-thalassemias, cri du chat syndrome and some retinoblastomas. The present process and reagents are applicable to detecting genetic disorders or variations primarily when a multi-base nucleotide deletion, insertion, substitution or transposition is involved in distinguishing the target nucleotide sequence from any other sequence present in samples intended to be read as negative for the target sequence. To the extent that the present invention is applicable to genetic disorders due to single base mutations, if at all, the complement of the substituted base or other point of mutation is desirably part of the target binding region of the probe polynucleotide, with the location of that base within the region likely to affect the selectivity of the method. Changes or differences in the expression, activation or rearrangement of structural genes, regulatory genes or oncogenes can be detected by the present method. For monitoring gene expression, messenger RNA could be targeted, assuming the expected activity in the present invention of RNA target nucleotide sequences. The present process can also be applied to histocompatability typing for tissue transplantation, determination of antibiotic resistance genes in microorganisms, and to the screening of food, medicinal and water samples for specific infectious agents or other microorganisms.

Selecting a target sequence for a particular test may involve determining a sequence which is unique or relatively unique to the target organism or condition. Such target sequence would be used to develop the target binding region as complementary thereto and then a labeled polynucleotide of appropriate length would be developed to bind to a part of the target binding region. In some instances multiple reagent complexes targeting different parts of the nucleic acid of the same organism or condition may be used in order to impart improved specificity when any particular target sequence is only relatively unique.

One embodiment of the present invention is illustrated, for purposes of understanding, by reference to attached Figures 1A—1E, in which is shown an immobilized probe polynucleotide P, 3300 nucleotides in length. Numbering from the 5' end, the region from nucleotide 3000 to nucleotide 3300 represents the target nucleotide binding region (TBR). For simplicity, it is assumed that the first nucleotide of the probe is directly attached to a support S. As illustrated in Figure 1A, the labeled polynucleotide L consists of 150 bases to which a tag T is attached at the 5' end. Of these 150 bases, 51 (from base number 100 to base number 150) bind specifically to the labeled polynucleotide binding region (LBR), bases 3250 to 3300, of the probe. Accordingly, when the immobilized probe consisting of probe polynucleotide P attached to support S is incubated with a solution of labeled polynucleotide L under normal hybridization conditions, a complex will be formed as shown in Figure 1A with labeled polynucleotide L attached at the end of the probe polynucleotide P via base pairing between bases 3250—3300 of the probe P and bases 100—150 of the labeled polynucleotide L.

In use, the illustrative complex is contacted with a biological sample under hybridization conditions in the presence of the recombination protein, with the sample containing a nucleotide sequence with bases 10000 to 10300 of this sequence which are complementary to and bind selectively to the target binding region TBR of the probe. Under proper hybridization conditions, the sample polynucleotide G could first bind, as illustrated in Fig. 1B, from base 10051 to base 10300 of G and base 3000 to base 3249 (the initial binding region or IBR) of probe P. The recombination protein, if it has hybridization-enhancing activity, would catalytically promote this step under appropriate conditions. In the presence of appropriate recombination protein, other regions of pairing, both in single-stranded and double-stranded regions are possible, such that such pairing may also occur at points within the labeled polynucleotide binding region LBR (such other forms of pairing are not shown in the Figures). The IBR in the reagent complex shown in Fig. 1A is entirely single stranded. It should be appreciated that thermodynamic considerations favor the formation of double-stranded moieties such as shown in Fig. 1B over single-stranded moieties. Thereafter, an equilibrium is created at bases 3250 to 3300 (LBR) of the probe polynucleotide P between binding to the labeled polynucleotide L and binding to bases 10000 to 10050 of the sample polynucleotide G. In the absence of recombination protein, a rapid zippering and unzippering will occur, as represented by the differences between Figure 1B on the one hand and Figure 1C on the other hand, along with random migration of the nucleic acid strand branch point along the probe polynucleotide. In such event, when additional bases of the sample polynucleotide G bind to the probe polynucleotide beyond probe base 3249, corresponding bases of the labeled polynucleotide L are displaced, creating a free end. In the absence of the recombination protein, this zippering and unzippering can occur in either direction; but the favored

13

event will be eventual shifting of the point of attachment to the right in Figure 1C. Figure 1D shows the stage of near total labeled polynucleotide strand displacement in somewhat enlarged and more graphic form from base number 3275 on the probe P toward base number 3300, at which point the labeled polynucleotide L is totally displaced from the probe P (cf. Figure 1E). It should be appreciated that, merely· upon random zippering and unzippering in both directions, removal of the labeled polynucleotide L will be favored in that Figure 1B represents the furthest to the left that the labeled polynucleotide L can displace the target nucleotide sequence of sample polynucleotide G. As long as some substantial region of the initial binding region IBR exists in which a sequence of nucleotides of the probe polynucleotide P binds selectively to the target nucleotide sequence, but not to the labeled polynucleotide, displacement of the sample polynucleotide G completely from the probe will be a rare and reversible event.

In the presence, however, of recombination protein, other mechanisms may also occur. In particular, the addition of *E. coli* rec A protein to the strand displacement reaction may enhance the overall release of the labeled polynucleotide by enhancing the rate of hybridization of analyte DNA to the initial target binding region IBR in the displacement complex; displacement of the labeled polynucleotide would then occur as described above. *E. coli* rec A protein, however, also catalyzes strand exchange reactions, and it is not clear at this time whether reactions are enhanced by rec A catalysis of just the initial hybridization event, by the additional catalysis of strand exchange between analyte DNA and reagent complex, or by both catalytic mechanisms.

It is not critical, however, to the present invention which mechanism(s) actually occur since the selection of precise reagent parameters (e.g., lengths of IBR and LBR) and reaction parameters (e.g., temperature and ion concentrations) may be similar in either event. Such parameters can still be determined empirically without undue experimentation in light of the known mechanisms available given the expected activities of the recombination protein.

An important parameter in optimizing the present method and reagent is the length and nature of the base pairing between the labeled polynucleotide and the probe polynucleotide. As compared to the 51 bases of exact pairing illustrated in Figure 1A, modification may be made either by shortening or increasing this length, by introducing mismatches or loops in either strand or by selecting between DNA and RNA for either or both strands so as to effect the rate of spontaneous dissociation. In this regard, certain differences can be achieved when the target nucleotide sequence is RNA and the labeled polynucleotide is DNA, whether the probe polynucleotide (P) is either DNA or RNA. This is because, in general, RNA—RNA binding is strongest per base pair, DNA—RNA base pairing is of intermediate strength and DNA—DNA base pairing is of the least strength per base pair. If, however, the target binding region (TBR) is larger than the labeled polynucleotide binding region (LBR), then the labeled polynucleotide can be RNA and still be displaced by a DNA target nucleotide sequence. Targeting RNA may be required if, for example, the target microorganism is an RNA virus or the target condition is one of altered gene expression. The beneficial effects of recombination proteins have been demonstrated to date, however, exclusively with DNA target nucleotide sequences. Greater or lesser enhancement may be achieved with RNA target nucleotide sequences, and the preferred recombination proteins may differ for RNA target nucleotide sequences.

One means of reducing the binding strength in the region in which the labeled polynucleotide is bound to the probe polynucleotide is the introduction of individual base mismatches into the labeled polynucleotide. Assuming that the probe nucleotide sequence will be chosen to pair exactly or nearly exactly to the target nucleotide sequence, such mismatches can be considered as mutations or individual base substitutions in the labeled polynucleotide compared to a similar polynucleotide segment of the target nucleotide sequence which has exact (or nearly exact) binding. Such mutation or substitution may include the substitution of a natural or chemically modified nucleotide for a given natural nucleotide such as the following: G for A (to produce the opposing pair dG-rU, dG-dT, rG-dT or rG-rU), A for G, 5-methylcytosine for C, 5-hydroxymethylcytosine for C, gentibiosyl-5-hydroxymethylcytosine for C, or 5-bromouracil for A. In many preferred embodiments, such mutations involve the substitution of one naturally occurring nucleotide for another nucleotide. In certain such embodiments, the substitution involves the substitution of a pyrimidine for a purine, leading to a mismatched pair that has become a pyrimidine-pyrimidine pair. Because pyrimidines occupy less space than purines, such individual pyrimidine-pyrimidine mismatches can have a minimal effect upon adjacent nucleotide pairings. Purine-pyrimidine mispairings (for example, A being positioned opposite to C, or T or U being positioned opposite to G) are somewhat more space filling, but are still generally less space filling than the positioning of two purines (A—A, A—G or G—G) apposite each other. Counteracting the space-filling effect, however, is a stacking energy effect which runs in the opposite direction: generally, purine/purine base pairs display a lower free energy of stacking, purine/pyrimidine base pairs somewhat more and pyrimidine/pyrimidine base pairs somewhat more still. *See* R. L. Ornstein & J. R. Fresco, *Biopolymers*, Vol. 22, pp. 1979—2016 (1983) (two articles). The net effect, in terms of effect on melting temperature of the reagent complex, in terms of effect on stability of the reagent complex and in terms of effect on the displacement kinetics, will primarily represent a combination of these two counteracting effects, such that experimentation may be required to determine which, if any, mismatches may be preferably incorporated into the labeled polynucleotide for a particular probe after the position of the target nucleotide region and labeled polynucleotide region are fixed.

In addition to point substitutions, mismatches can be created either by inserting a short sequence in the labeled polynucleotide L which does not correspond to the probe nucleotide sequence (e.g., inserting a series of A's between bases 125 and 126 of labeled polynucleotide L in the example illustrated in Figure 1A) or by deleting a portion (such as by deleting bases 120—130 of labeled polynucleotide L in Figure 1A). Insertions in the labeled polynucleotide will generally form a single-stranded loop or cruciform structure of the labeled polynucleotide; deletions therein will generally form a single-stranded loop or cruciform structure of the probe polynucleotide.

Such substitutions, deletions or insertions will have the effect of destabilizing the binding between the labeled polynucleotide L and the probe polynucleotide P -such that the displacement of the labeled polynucleotide may be favored. It is important, however, to avoid to the extent possible nonspecific displacement of the labeled polynucleotide L from the probe polynucleotide P in the absence of the target nucleotide sequence. The minimum length of binding between the labeled polynucleotide L and the probe polynucleotide P which is sufficient to minimize such dissociation or falling off will be dependent on a number of factors including, especially, the conditions such as pH and temperature of hybridization, the mode of attachment of the probe polynucleotide to the support (e.g., end attachment versus nonspecific adsorption), the extent of destabilizing substitutions (or deletions or additions), the duplex base sequence at the region of hydrogen bonding and whether the binding is between RNA and RNA, DNA and RNA or DNA and DNA. The optimal conditions in a particular instance can be determined empirically with routine experimentation based upon the general teachings of the present disclosure. Such experimentation may involve melting temperature experiments with samples lacking the target nucleotide sequence for purposes of estimating stabilities, and then displacement experiments for final optimization.

In considering the geometric relationship between the region where the labeled polynucleotide binds to the probe polynucleotide (LBR) and the region where the target nucleotide sequence binds to the probe polynucleotide (TBR) a configuration such as that illustrated in Figures 1A—1B may be used, with the labeled polynucleotide binding region being a subset of and at the end of the target binding region distal to the solid support. A common end of the TBR and the LBR (probe nucleotide 3300 in Figure 1A) may also be (or be near to) one end of the probe polynucleotide. There are, however, no great disadvantages in having the probe polynucleotide extend beyond this point in a sequence of a nonspecific nature. There may be situations in which it is desirable to have a probe polynucleotide continue beyond this pairing region and extend to a point of attachment of a tag (different from the tag T on labeled polynucleotide L) which is to be released subsequently by other techniques. Furthermore, the two tags (one on the labeled polynucleotide, the other in the probe polynucleotide) may interact, with the interaction being detected as a part of the read-out. It may be preferred in certain embodiments of the invention that the labeled binding region be near or at the end of the target binding region nearest the support.

Various other geometries suitable for practice of the present method, either by a displacement reaction from a supported probe or by a displacement reaction in solution, are illustrated in Figures 1F, 1G, 2, 3A, 3B, 3C, 3D, 4, 5 and 6. The effect of recombination protein on these geometries should be similar to what is described above for Figures 1A—1E.

Situations which are included in the present invention, but which may be less preferred include extension of probe polynucleotide P beyond the LBR (that is, for example, for the 125 bases beyond base 3300 in Figure 1F) with a region (such as bases 3375—3425) which binds selectively to a portion of the sample nucleotide (and especially a region such as bases 9750—9800 of the sample polynucleotide G). In such an event, the sample polynucleotide G could bind both at bases 3000—3249 of the probe polynucleotide P and at bases 3450—3500 of the probe polynucleotide P and overlay the labeled polynucleotide L. Such a structure, represented generally as a "triple-stranded" region or D loop (see C. Green and C. Tibbetts, *Nucleic Acids Research*, vol. 9, No. 8, pp. 1905—18 (1981), especially page 1912), might not cause displacement of the labeled polynucleotide L in certain topologies and thus would represent a potential specific binding event without a signal generation. While displacement at bases 3250—3300 of the probe might still be possible (and might even proceed from both ends), the loss of free movement of the sample strand G due to binding at its bases 9750—9800 may reduce the probability of complete displacement, especially when one considers the helical structures actually involved.

Another situation within the scope of the present invention, but also somewhat less preferred than the above, is that in which the labeled polynucleotide binding region (LBR) extends beyond and outside the target binding region (TBR). This second embodiment of the invention is illustrated in Figure 1G (and in Examples 13 and 14), wherein the reagent complex is shown, with the target nucleotide sequence being capable of displacing the labeled polynucleotide as far as the end of the TBR. The labeled polynucleotide would then remain bound via those probe bases which are part of the labeled polynucleotide binding region LBR but not part of the target binding region TBR (such sequence of bases being designated the residual binding region or RBR). Provided that the RBR (e.g., bases 3300 to 3305 of probe P in Figure 1G) is much smaller than the LBR, one could, in theory, change conditions at this point (pH or temperature for example) so as to totally release the labeled polynucleotide L in this complex without releasing other labeled polynucleotides bound to the entire labeled polynucleotide sequence LBR (see Example 13 where this is described for a displacement in solution). One should consider, however, that the hybrid shown in Figure 1G will still be capable of displacement to the left after complete binding of a target nucleotide sequence G, with the labeled polynucleotide capable of displacing part or all of the portion of the target polynucleotide sequence bound to bases within the overlap of TBR and LBR; such an event could be expected to lower the efficiency of displacing the labeled polynucleotide.

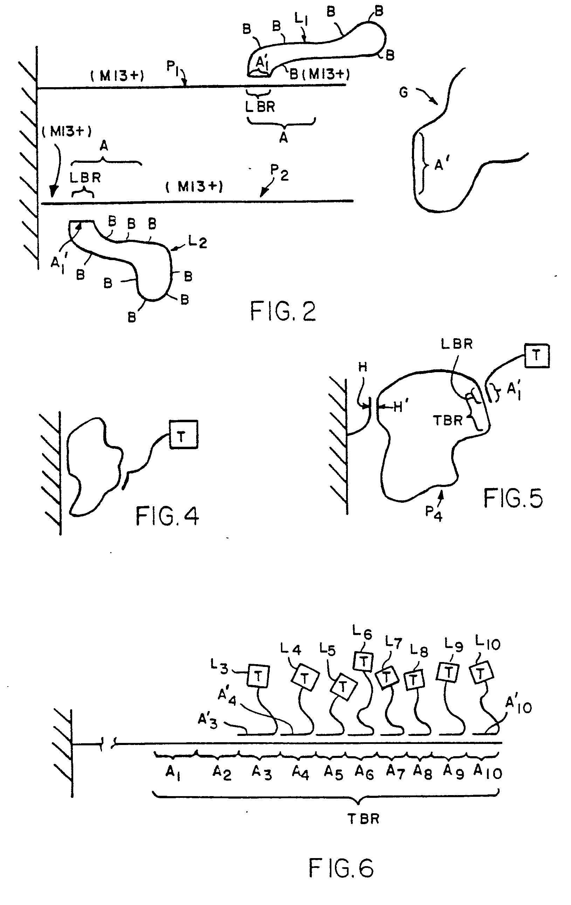

Other embodiments of the invention different from those illustrated in Figures 1A through 1G are illustrated in Figures 2—6 and discussed briefly below.

Figure 2 illustrates a third embodiment of the invention using a complex different from that of Figure 1A in that the labeled polynucleotide $L_1$ is the circular plus (or infectious) strand (M13$^+$) of DNA bacteriophage M13 into which is inserted a segment $A_1'$ complementary to a portion (LBR) of the target binding region A of the probe strands $P_1$ and $P_2$. The label in this embodiment is shown as a series of biotin moieties B distributed within the M13$^+$ major portion of the labeled polynucleotide $L_1$, possibly by growing M13 in bacterial culture with biotinated nucleotide triphosphate (e.g. biotinated—dATP) present or by chemically attaching biotin afterwards to the M13 nucleotides. The $A_1'$ segment may also contain nucleotides bound to biotin. The sample polynucleotide G will, in this instance (Figure 2), contain a target nucleotide sequence A' complementary to segment A of each probe $P_1$ and $P_2$. In this embodiment the probe polynucleotides may also be formed using bacteriophage M13, with segment A complementary to the target nucleotide sequence A' inserted at a location in the non-essential region of the M13 genome. The M13$^+$ segments of the probe are non-complementary (actually equal or homologous) to M13$^+$ regions of the labeled polynucleotide. If the circular phage is cleaved non-specifically (e.g., by partial cleavage with restriction enzyme Hae III; c.f. R. W. Blakesley and R. D. Wells, *Nature*, vol. 257, pp. 421—422 (1975)), linear strands will be created having segment A positioned at different points relative to the ends, but generally with a portion of the M13$^+$ strand on both sides of segment A. This is illustrated in Figure 2 by strand $P_1$, wherein the segment A is close to the free end of supported polynucleotide, and strand $P_2$, wherein segment A is close to the attached end. Of course, cleavage will also potentially produce shortened strands (due to two or more cleavages in the M13$^+$ region) and could also produce strands with region A split between the two ends (due to cleavage in region A); the latter condition can result in failure to form a complete and fully operative target binding region. So long as region A is small relative to the length of the M13$^+$ strand (7.2 kilobases; J. Viera & J. Messing, *Gene*, vol. 19, 259—68 (1982)), relatively few probe strands would be expected to have region A split. Uniformity in location of segment A can be achieved by cleaving single-strand M13 DNA specifically (e.g., with a restriction enzyme recognizing an inverted repeat sequence in or inserted into the M13 genome).

After displacement, the biotinated displaced labeled polynucleotides of Figure 2 or related embodiments could be concentrated from the liquid phase, e.g., with a strepavidin column, before readout using, e.g., horseradish peroxidase linked to strepavidin and colorimetric, fluorimetric or chemiluminescent readouts based upon the peroxidase activity.

Figure 3A illustrates a fourth embodiment of the present invention with the reagent complex entirely in solution (no support is present). The probe polynucleotide P has a target binding region (TBR) including a subregion, the labeled polynucleotide binding region (LBR), in which bases are bound to complementary bases of the labeled polynucleotide (L). The labeled polynucleotide (L) contains a fluorescent tag (F) held in the complex in close proximity to a quencher moiety (Q) attached to the probe (P). These moieties F and Q are sufficiently close in the complex of Figure 3A for any signal produced by stimulation of the fluorescent tag (F) by radiation to be absorbed by the quencher moiety (Q). An exemplary pair of F and Q are fluoroscein with rhodamine. See M. Cobb and S. Gotcher, *Am. J. Med. Tech.*, Vol. 48, No. 8, pp. 671—677 (1982).

Figure 3B illustrates the reagent complex of Figure 3A after contact by a sample G containing the base sequence complementary to the entire target binding sequence (TBR). As in Figure 1B, binding will be first to the unpaired region of TBR and then displacing portions of the labeled polynucleotide L from the subregion LBR. The stage of partial displacement (such as seen in Figures 1C and 1D) is illustrated in Figure 3B. Upon completion of the displacement, the target sequence of sample polynucleotide G will be bound to the entire region TBR of probe P and the labeled polynucleotide L will be totally displaced. In this situation, as illustrated in Figure 3C, the tag (F) is now sufficiently far from quencher moiety (Q) to produce a detectable signal on stimulation that is not quenched (except in those rare cases where another probe P happens to be located such that its label Q is in close proximity to tag F). Accordingly, the stimulation of fluorescent tag (F) and measurement of unquenched signals can serve as a quantitative detection method for displaced labeled polynucleotides without a separation step having been performed.

A modification in the embodiment of Figures 3A—3C in which the labeled polynucleotide L and probe polynucleotide P (bearing, respectively, labels F and Q) are part of the same molecule is illustrated in Figure 3D. In addition, a site X is shown which contains an inverted repeat sequence a portion of which is recognizable by a restriction enzyme. Cleavage by such an enzyme can be used to sever the covalent linkage between TBR and L (although this severing is not required when the read-out is based upon the F—Q interaction). Such cleavage can also be used as a first step in creating a site for attachment of the probe to a support, such attachment to occur before or after displacement. The overall geometry of Figure 3D may thus be used in synthesizing reagent complexes as a single molecule for various embodiments, including those of Figures 1A—1E.

Figure 4 illustrates a complex in which the probe strand $P_3$ is a circular single-stranded polynucleotide non-specifically adsorbed on support S. Such a polynucleotide can be produced by insertion of a target binding region (TBR), into a single-stranded bacteriophage such as M13 through suitable cloning procedures. The labeled polynucleotide L here contains a segment $A'_1$ complementary only in a portion LBR of segment TBR.

Figure 5 illustrates a complex similar to Figure 4 except that, instead of being adsorbed to support S, probe strand $P_4$ is linked via a linking polynucleotide covalently attached to support S. Such linking polynucleotide contains a region H complementary to a segment H' of the probe $P_4$ remote from the TBR segment.

Figure 6 illustrates a complex similar to the embodiment of Figure 1A in that the probe strand $P_5$ contains a target binding region TBR near or at its free end. A number of portions of the TBR segment (subsegments $A_3$, $A_4$, $A_5$, $A_6$, $A_7$, $A_8$, $A_9$, and $A_{10}$) are each base paired to a complementary region of a labeled polynucleotide; segment $A'_3$ of labeled polynucleotide $L_3$ to subsequent $A_3$, etc. Probe $P_5$ contains a target binding region TBR divided into subsegments $A_1$ and $A_2$, together forming the initial binding region, as well as eight additional subsegments ($A_3$ thru $A_{10}$), each forming a labeled polynucleotide binding region. In this embodiment, binding of segment A' of the sample polynucleotide G (as shown in Figure 1E) will displace all of the labeled polynucleotides $L_3$, $L_4$, $L_5$, $L_6$, $L_7$, $L_8$, $L_9$ and $L_{10}$ and thus produce an enhanced signal compared to the situation shown in Figure 1A. It should be appreciated, however, that the absence of any of those labeled polynucleotide (e.g., $L_8$) could permit the target nucleotide to form D-loops (as in Figure 1F) by binding to subsegments $A_1$ and $A_2$ (for which no labeled polynucleotide is complimentary) and any other probe subsegment (e.g., $A_8$) for which the labeled polynucleotide is absent on that particular probe strand.

Examples

In certain of the following examples, four small synthetic DNA oligomers were used as the tagged polynucleotides; all were labeled with radioactive phosphorous-32 atoms. Two oligomers were 27 base oligomers matching 26 or 27 bases at the 3' end of one strand of a 1.1 kb pBR322 DNA fragment. This fragment stretched from the Pst I restriction site to the Bam HI restriction site in pBR322 DNA. These two oligomers (L1 and L2), and the labeled polynucleotide binding region to which they were bound, are illustrated in Table 1. The mismatched pyrimidine base in oligomer L2 is underlined.

TABLE A

```
                                    350
Probe 5' end...    T—A—C—G—C—G—A—T—C—A—
L1                           3'C—T—A—G—T—
L2                           3'C—T—A—G—T—
```

```
                        360                    367
Probe   T—G—G—C—G—A—C—C—A—C—A—C—C—C—
L1      A—C—C—G—C—T—G—G—T—G—T—G—G—G—
L2      A—C—C—G—C—T—G—G—T—G—T—G—G—C—
        20                          10   9
```

```
        370                        380
Probe   G—T—C—C—T—G—T—G—G—A—T—C—C...3'end
L1      C—A—G—G—A—C—A—C5' end
L2      C—A—G—G—A—C—A—C5' end
```

Example 1

An 840 base pair Eco RI-Bgl II human DNA restriction fragment containing the first 262 codons of the human serum albumin gene was subcloned into the single-stranded bacteriophage vector M13mp9, generating the clone designated M13mp9-albumin (see Lawn et al., *Nucleic Acids Research*, vol. 9, pp. 6103—6114 (1981) for the DNA sequence of the human albumin gene). Also inserted into this vector was the Eco RI fragment containing the polylinker from M13mp7. This polylinker sequence, inserted adjacent to the albumin sequence at the Eco RI site, is capable of forming a double-stranded hairpin structure in the otherwise single-stranded molecule. Cleavage of such a molecule with the enzyme Bam HI resulted in a linear and full length single-stranded molecule. An end-labeled complementary polynucleotide to this full-length molecule was prepared by kinasing a 14 base long oligonucleotide (5' end-GATGCACACAAGAG-3' end) with P-32-labeled ATP and subsequently hybridizing it to the albumin insert before extending the labeled polynucleotide *in vitro* using *E. coli* DNA polymerase. The resulting DNA molecules were digested with Pvu II, which cuts once in the human albumin insert. The final labeled polynucleotide, a labeled 175 base long single stranded molecule, was then purified on a denaturing agarose gel by electrophoresis. The purified labeled polynucleotide was hybridized to the above full-length M13mp9-albumin DNA strand which serves as the probe polynucleotide; the probe used was either circular or had been linearized with Bam HI. Unhybridized labeled polynucleotide was removed from the reagent complex by passage over two successive Sepharose® CL-4B columns.

# 0 164 876

Model competitor DNAs used in strand displacement experiments were either the complementary DNA strand to the human albumin sequence of the M13mp9-albumin clone inserted in the vector M13mp8 (and hereafter designated as M13mp8-albumin), or a denatured double-stranded 840 base pair Eco RI-Bgl II human DNA fragment which is colinear with the insert in the M13mp9-albumin clone. The displacement reactions were carried out by incubating approximately 10 nanograms of the reagent complex containing a circular or linear probe polynucleotide with 10 or 50 nanograms of competitor DNA. All reactions were done in solutions containing 20mM Tris-HCl, pH7.5, 25 mM $MgCl_2$, 0.1 mg/ml BSA, 0.3mM DTT, and, when included, 8mM ATP (cf. S. West et al., *Proc. Nat. Acad. Sci. U.S.A.*, vol. 78, pp. 2100—2104 (1981)). Reaction mixtures containing either no *E. coli* rec A protein, 5 picomoles of rec A protein or 10 picomoles of rec A protein (*E. coli* rec A protein having been purchased from PL Biochemicals) were incubated for 60 minutes at 37°C. The reactions were stopped and the DNA deproteinated by adding chemical reagents to raise the final sample solution concentrations to 20mM EDTA, 0.4% SDS and 0.4 mg/ml proteinase K and incubating at 37°C for an additional 30 minutes. The amount of displacement was assayed by separating the reagent complex from displaced strands on a 2% agarose gel by electrophoresis, and autoradiographing the gel overnight.

When a displacement reaction was done using reagent complex with a circular probe polynucleotide and M13mp8-albumin DNA but without the addition of rec A protein under the conditions described above, no detectable displacement of the labeled polynucleotide from the probe was observed. The addition of up to 10 pmol of rec A protein to the reaction between the circular reagent complex and the circular single stranded DNA competitor resulted in the displacement of approximately 5% of the probe. However, most of the reagent complex was shifted to a position of low mobility on the gel, indicating that rec A protein may have promoted the association of the competitor DNA and the reagent complex. This suggested that topological constraints could have prevented the displacement reaction from going to completion. When linearized reagent complex was used, the addition of 10 picomoles of rec A protein was estimated visually by examination of the autoradiograph to result in a displacement of approximately 75% of the release tag from the probe using circular competitor DNA. Less displacement was observed when 5 pmol of rec A was used, and increasing the amount of the competitor DNA to a 5-fold molar excess resulted in no observable increase in displacement, suggesting that the action of rec A protein is limiting under these conditions. Displacement was also observed when the double-stranded linear 840 base pair human DNA fragment was denatured and used as competitor DNA in a similar experiment to the above. A 10-fold molar excess of this competitor when linearized resulted in the displacement of only about 10% of the labeled polynucleotide even in the presence of rec A protein. Increasing the competitor DNA concentration to a 40-fold molar excess resulted in displacement of up to approximately 40% of the labeled polynucleotide. It is likely that this reaction was less efficient (with this embodiment of separation and detection) than strand displacement induced by single-stranded competitor DNA due to interferences from reannealing of the two self-complementary strands of the competitor DNA (or of the inactive competitor strand with displaced labelled polynucleotide).

## Example 2

A reagent complex was prepared and purified as described in Example 1 using the mp9albumin-polylinker template and the labeled 175 nucleotide primer-extended DNA described in Example 1, except that the circular probe DNA of the reagent complex was not cleaved with Bam HI. All displacement reactions were done in 20mM Tris, pH 7.5, 25 mM $MgCl_2$, 0.1 mg/ml BSA, and 0.3 mM DTT in a final volume of 25 ul. The ATP concentration was varied in this experiment to determine the effect of this parameter. After the addition of approximately 10 ng of circular M13mp8-albumin DNA (analyte) and, when included, 5 pmol of the rec A protein the reaction mixtures were incubated for 60 minutes at 37°C. Reactions were stopped and the DNA deproteinated as described in Example 1 and electrophoresed on a 1.5% agarose gel to assess displacement. Incubation of the reagent complex in the absence of rec A protein, ATP and competitor resulted in no displacement demonstrating the stability of the reagent complex under these conditions. Incubation of 10 ng of reagent complex with 10 ng of competitor DNA (approximately a 1:1 molar ratio) in the absence of rec A protein resulted in no strand displacement under these conditions. The addition of 5 pmoles of rec A protein but no ATP to the reaction also resulted in no displacement. When reaction mixtures containing rec A protein were adjusted to either 5mM or 10 mM ATP, approximately 50% of the radiolabel was estimated visually to be at a position of slower mobility on the gel, suggesting that association of the circular reagent complex with the circular competitor DNA had occurred. In addition, a small amount (5%) of displacement was observed in these reactions with ATP. As described in Example 1, topological constraints on two circles hybridizing may inhibit the displacement of labeled polynucleotide molecules from the reagent complex. When the concentration of ATP was increased to 20 or 40 mM in certain samples, no strand displacement was observed. This inhibition may be due to low levels of ADP contamination in the ATP preparation, or to rapid accumulation of ADP in the sample from hydrolysis of ATP by rec A protein. Analogous ADP inhibition of rec A activity has been previously described in M. Cox et al., *J. Biol. Chem.*, vol. 258, pp. 2586—2592 (1983); see also Shibata et al., *J. Biol. Chem.*, vol. 256, pp. 7565—7572 (1981).

18

Example 3

A reagent complex was prepared using the linearized reagent complex described in Example 1. The model competitor DNA (M13mp8-albumin DNA) was again the polynucleotide strand complementary to the human albumin insert cloned into M13 (part of the reagent complex). All displacement reactions were carried out essentially as in Example 2, except that the amounts of analyte DNA, ATP, and rec A protein as well as the time period of incubation were varied. Approximately 10 ng of reagent complex were used in each reaction sample. After the addition of ATP, competitor DNA (equimolar or 1×, or 10-fold molar excess or 10×) and rec A protein, samples were incubated at 37°C for 15, 30 or 60 minutes. Reactions were stopped and DNA deproteinated as described in Example 1. Samples were then electrophoresed on a 1.5% agarose gel, which was subsequently dried and autoradiographed. Amounts of displacement (Table 1) are estimates based upon visual analysis of certain autoradiograph lanes. The observations for other lanes were also consistent with progressive strand displacement over the course of the 60 minute incubation period.

TABLE 1

| Sample | Competitor | p moles rec A | ATP (mM) | Time min | % Cpm displaced |
|--------|-----------|---------------|----------|----------|-----------------|
| 1 | 0 | 0 | 0 | 60 | 1 |
| 2 | 10X | 0 | 0 | 15,30 60 | 1 |
| 3 | 1X | 5 | 5 | 15 | 5 |
| 4 | 1X | 5 | 5 | 30 | 25 |
| 5 | 1X | 5 | 5 | 60 | 40 |
| 6 | 1X | 20 | 5 | 60 | 40 |
| 7 | 1X | 10 | 20 | 60 | 5 |
| 8 | 5X | 10 | 10 | 60 | 60 |
| 9 | 5X | 20 | 10 | 60 | 80 |
| 10 | 10XNS | 10 | 10 | 60 | 2 |

The data indicate that, in the absence of rec A protein, there was no detectable strand displacement of the labeled polynucleotide with a tenfold molar excess of analyte DNA under these conditions. The addition of rec A protein to reaction mixtures containing equimolar amounts of analyte and reagent complex resulted in the displacement of up to approximately 40% of the labeled polynucleotide over the 60 minute incubation. Increasing the amount of rec A protein from 5 pmoles to 20 pmoles resulted in no significant increase in the amount of labeled polynucleotide displaced during a 60 minute incubation. Increasing the ATP concentration from 5 mM to 10 mM resulted in no change in displacement, but an increase to 20 mM ATP apparently inhibited the displacement reaction (possibly due to ADP build-up).

The addition of 50 ng of analyte DNA (5% molar excess) to about 10 ng of reagent complex in the presence of 10 mM ATP and 10 pmoles of rec A protein resulted in the displacement of approximately 60% of the labelled polynucleotide (see Table 1). Increasing the amount of rec A protein to 20 pmoles resulted in an increased displacement up to approximately 80% of the labeled polynucleotide. No displacement was observed when 50 ng of bacteriophage M13 mp7 DNA (5× of a non-specific competitor DNA; "NS" in Table 1) was used in a reaction with 10 mM ATP and 10 pmoles of rec A.

Example 4

The probe used in this experiment (M13mp8pBR6PL6) was prepared by subcloning the 650 base pair Eco RI-Sal I fragment from pBR322 into the M13mp8 vector. Also inserted into this bacteriophage at the Eco RI site is the polylinker from M13mp7 which is a small inverted repeat containing several restriction enzyme cleavage sites. Single stranded circular M13mp8pBR6PL6 was isolated and digested with Bam HI, an enzyme which cuts within the hairpin loop of the polylinker and linearizes the single stranded DNA. A $^{32}$P end-labeled polynucleotide was synthesized by kinasing an oligonulceotide which is complementary to the insert in mp8pBR6PL6. Primer extension and cleavage with EcoRI resulted in a 350 base end-labelled DNA fragment extending from the BamHI site to the EcoRI site in mp8pBR6PL6. This labeled polynucleotide was then purified by electrophoresis on a denaturing agarose gel.

The reagent complex was made by hybridizing the labeled 350 base polynucleotide to the BamHI linearized mp8pBR6PL6 probe; hybridized complex was purified away from unhybridized probe by two consecutive passages over a Sepharose® CL-4B column. The complementary strand of the Eco RI-Sal I fragment was inserted into the M13 vector mp11 and single stranded DNA from this recombinant phage (mp11pBR12) was used as a model competitor DNA.

Strand displacement reactions were carried out essentially as in Example 1, except that all reaction mixtures contained 10 mM ATP. In addition, after all components of the reaction except competitor DNA were mixed, varying amounts of E. coli rec A protein were added and allowed to preincubate with the displacement complex at 37°C for 2 minutes. Competitor DNA was then added and the reactions proceeded until they were halted after 15 or 60 minutes (only these two times of incubation were assayed) and were deproteinated as described in Example 1. The addition of 10 ng of competitor DNA to a displacement reaction mixture containing an equimolar amount of reagent complex resulted in the displacement of approximately 20% of the labeled polynucleotide in 60 minutes in the presence of 15 or 30 pmoles of E. coli rec A protein. Less than 2% displacement was observed in the absence of E. coli rec A protein under the same conditions. When 50 ng of analyte DNA (5-fold molar excess over reagent complex) were added in the presence of 15 or 30 pmoles of rec A protein, approximately 50% displacement was observed after 60 minutes. Less displacement was observed after 15 minutes of incubation for all reactions. The displacement from this reagent probe complex may be less efficient than that in Examples 2 or 3 due to one or more of the several differences in the geometry of the several reagent complex constructs (involving different absolute and relative lengths of initial binding region, different positioning of the labeled polynucleotide binding region at the 5' end of or interior of the target binding region or the size of the labeled polynucleotide binding region). It was in limited parallel runs to those of this Example 4 that the addition of single-stranded binding protein from E. coli (0.5 pmoles) appeared to inhibit the appearance of displaced labeled polynucleotide mediated by E. coli rec A protein.

Example 5

The M13mp9 bacteriophage recombinant molecules used in this and the following Examples 6—14 as probe DNA (M13mp9 clone II-16 DNA) had a 1.1 kb nucleotide insert (PstI-BmmHI fragment) from pBR322 oriented with the Bam HI site located in the 3' direction relative to the PstI site. In certain experiments the circular M13mp9 clone II-16 DNA was linearized and fragmented before hybridization; this was done by incubating the DNA with the restriction enzyme Hinfl (about 2 units per ug DNA) at 37°C for 3—4 hours. Analysis of product material from such digestions on an agarose gel showed that a number of fragments was produced as expected from the multiple recognition sites in M13 for Hinfl (cf. R. D. Wells and S. K. Neuendorff in *Gene Amplification and Analysis*, vol. 1: *Restriction Endonucleases*, pp. 101—111 (Elsevier Press, N.Y. (1981)). The labeled polynucleotide used in this Example is one completely complementary to the 27 nucleotides at the 3' end of the pBR322 insert in M13mp9 clone II-16 (i.e., adjacent to the BamHI recognition site). The nucleotide sequence of the region of the probe DNA which is completely homologous to the labeled polynucleotide and the sequence of the labeled polynucleotide are shown in Table A above (probe and oligomer L1). The 27-mer polynucleotide was ratioisotopically end-labeled with $^{32}$P using T4 polynucleotide kinase. Unincorporated gamma-[$^{32}$P] ATP was then removed from labeled oligonucleotide preparation by chromatography on a Whatman DE52 column.

A DNA-DNA hybrid (reagent complex) was formed between the 27 nucleotide oligomer (labeled polynucleotide) and the M13mp9 clone II-16 recombinant DNA (probe molecule). Hybridization to produce this reagent complex was carried out by mixing the probe DNA with a molar excess (usually five fold) of labeled polynucleotide in 5× SSC (750 mM sodium chloride. 75 mM sodium citrate). This mixture was incubated at 50—55°C for 15—50 minutes before reagent complex was then separated from unhybridized labeled polynucleotide on a Sepharose® 4B column. After preparing and purifying reagent complex DNA, simulated sample or analyte DNA containing target nucleotide sequences was introduced into a nucleic acid strand displacement reaction mixture under varying conditions as described below. The analyte DNA (also referred to as competitor DNA in this Example and subsequent Examples) was a circular single stranded DNA competitor. M13mp8 clone 20 DNA, which had a DNA insert consisting of the complementary strand to the pBR322 insert in bacteriophage M13mp9 clone II-16 as discussed above. A set of parallel strand displacement reaction samples were prepared in 11.6 µl rec A buffer solution (100 mM Tris-HCl, pH 7.5, 25 mM MgCl$_2$, dithiothreitol, ATP and 500 µg/ml bovine serum albumin) using reagent complex and competitor DNAs in a 1:1 molar ratio. One sample received 30 pmoles rec A protein or about forty times the amount of reagent complex on a weight basis. Incubation was at 37°C for 2.5 hours; an unincubated control was mixed and held on ice prior to analysis. Following incubation, the reaction mixtures were separated by electrophoresis on a 15% polyacrylamide gel to distinguish unbound (free and presumably displaced) labeled polynucleotide from any reagent complex molecules. The gel was autoradiographed and the distribution of $^{32}$P label in the resolved sample bands was determined by densitometry using a Shimadzu CS930 plate scanner.

In the absence of rec A protein and without circular competitor DNA, less than 6% labeled oligonucleotide was displaced from reagent complex (free) irrespective of whether or not the sample was incubated at 37°C for 2.5 hours. When competitor DNA in equimolar amount to reagent complex was present, 23% of the 27-mer was competed off when no rec A protein was included, but 70% was displaced in the presence of rec A protein during incubation. Thus, rec A protein can increase the extent of unbound polynucleotide observed following strand displacement occurring due to the presence of specific target polynucleotides.

Comparing Example 5 (and those Examples that follow) with Examples 1—4, one can see a demonstration of the beneficial effects of *E. coli* rec A protein with both long (up to 275 base) and short (27 base) labeled polynucleotide binding regions. In addition, in Example 5 (and those that follow) no proteinase digestion step was conducted at the conclusion of the displacement reaction.

A substantially similar experiment comparing the 15% polyacrylamide gel to an 0.8%/2.5% agarose gel gave similar results.

Example 6

Example 5 compares polynucleotide strand displacement at 37°C with and without rec A protein using two different types of analytical gel. Another experiment was conducted using the reagent complex preparation and the reaction conditions of Example 5 to examine the need for functional *E. coli* rec A in such strand displacement reactions and to compare strand displacement at 37°C using rec A protein to displacement under more conventional nucleic acid hybridization conditions (55°C in 2× SSC). The latter temperature is approximately $T_m-13°C$ for the reagent complex and is consistent with theoretical prediction of a temperature at or near the optimum for effective DNA—DNA strand hybridization in a simple salt buffer. Various control reaction mixtures (samples 1—4 and 6—7) showed that the proportion of unbound labeled polynucleotide was in the range of 20—25% when competitor DNA was omitted for all buffers tested except rec A buffer. The use of rec A buffer with competitor DNA raised the observable level of unbound oligonucleotide to 47%, for reasons not completely understood. At 55°C with competitor DNA in 2× SSC, 96% of the label was found as free 27-mer while 77% of all label was in the unbound 27-mer form following competitive displacement in the presence of rec A protein in rec A buffer at 37°C.

Example 7

An Example was sought which might demonstrate a concentration effect for *E. coli* rec A protein. In most Examples described herein, there was a 40-fold excess by weight of rec A protein over reagent complex or a molar ratio of about 3000 protein molecules: 1 DNA molecule (equivalent to about one protein molecule for every 3 nucleotides). Reagent complex was prepared and a set of displacement reactions was run in rec A buffer solution with and without rec A protein and/or polyethylene glycol (PEG), MW6000 (see Example 5 for details). Reagent complex containing linear M13mp9 clone II-16 probe polynucleotide was used with circular M13mp8 clone 20 competitor DNA. After all reaction mixtures were incubated at 40°C for 2.5 hours, samples were analyzed by electrophoresis on 15% polyacrylamide gels and subsequent autoradiography of the gels.

TABLE 2

| Reaction samples | Percent unbound 27-mer |
|---|---|
| 1. Rec A buffer solution, no competitor DNA* | 6 |
| 2. Rec A buffer solution | 44 |
| 3. Rec A buffer solution, 5× weight excess of rec A protein | 54 |
| 4. Rec A buffer solution, 40× weight excess of rec A protein | 85 |
| 5. Rec A buffer solution, PEG (MW 6000) | 43 |
| 6. Rec A buffer solution, 5× weight excess of rec A protein, PEG (MW 6000) | 81 |
| 7. Rec A buffer solution, 5× weight excess of rec A protein, PEG (MW 6000) | 89 |
| 8. Rec A buffer solution, 40× weight excess of rec A protein, PEG (MW 6000) | 73 |

*All other reaction mixtures had competitor DNA present in equimolar amount.

The results (Table 2) reveal that *E. coli* rec A protein alone or in conjunction with PEG promotes efficient strand displacement. Rec A protein alone in a 5-fold weight excess does show some ability to

21

enhance displacement under these conditions but the effect is significantly less than that seen with a 40-fold weight excess. Although PEG (MW 6000) alone shows no effect at this temperature, it does appear to act synergistically with a 5× weight excess of rec A protein. These results have been studied at both lower and higher concentrations of rec A protein in other related experiments and the conclusions are consistent to those reported here. An enhancement of strand displacement can be demonstrated with as little as a 3.4-fold weight excess of rec A protein under the conditions described herein. A 400-fold weight excess of rec A protein was not substantially better than a 40-fold weight excess under these conditions.

Example 8

Five reaction mixtures with different contents were prepared and incubated at 37°C as described in Example 5; samples for analysis were removed at 30 minute intervals beginning at 0 minutes and ending at 2.5 hours. Both the probe polynucleotide in the reagent complex and the competitor DNA were linearized with Hinf I. The concentration of *E. coli* rec A protein was a 40-fold weight excess over the reagent complex in all samples.

TABLE 3

| | Incubation temperature | Time (hours) | | | | | |
|---|---|---|---|---|---|---|---|
| | | 0 | 0.5 | 1 | 1.5 | 2 | 2.5 |
| 1. Rec A buffer solution, no competitor DNA* | 37°C | 3 | 3 | 0 | 6 | 5 | 10 |
| 2. Rec A buffer solution | 37°C | 4 | 5 | 7 | 2 | 5 | 10 |
| 3. Rec A buffer solution, 40× weight excess of rec A protein | 37°C | 4 | 43 | 69 | 76 | 74 | 79 |
| 4. 1× SSC | 37°C | 12 | 15 | 11 | 16 | 12 | 13 |
| 5. 1× SSC | 55°C | 6 | 29 | 55 | 62 | 71 | 65 |

*All other reaction mixtures had competitor DNA present. Results are expressed in percent unbound 27-mer.

The data (Table 3) obtained from autoradiograms prepared for these samples as detailed in Example 5 show that there is substantial free labeled 27-mer present only in samples incubated at 37°C with rec A protein or at 55°C in 1× SSC, both samples requiring competitor DNA be included. Maximal strand displacement is achieved by about 1.5 hours for each of these two conditions.

Example 9

A separate experiment demonstrated that the enhancement of strand displacement reactions with rec A protein depends on the functional activity of the protein since either boiling or absence of incubation removed any enhancement. Hence most reactions in the Examples were run at 37°C. Further study of any temperature dependence was sought by comparing strand displacement at 44°C with that at 37°C, using circular competitor DNA and circular probe polynucleotide in the reagent complex. Forty fmoles of reagent complex prepared as in Example 5 and forty fmoles of circular competitor DNA were combined in each reaction mixutre. Rec A protein was then added to a 40-fold weight excess relative to reagent complex before each sample was split into two portions. One half of each reaction mixture was incubated at 37°C and the other half at 44°C prior to analysis of free oligonucleotide (see Example 5 for details).

**0 164 876**

TABLE 4

| Reaction samples | Percent Unbound 27-mer | |
| --- | --- | --- |
| | 37°C | 44°C |
| 1. 2× SSC, no competitor DNA; unincubated control* | 6 | 9 |
| 2. 2× SSC buffer | 7 | 63 |
| 3. Rec A buffer solution | 8 | 100 |
| 4. Rec A buffer solution, 40× weight excess of rec A protein | 17 | 100 |

*All other reaction mixtures had competitor DNA and were incubated for 2.5 hours.

These data (Table 4) show a very dramatic effect of raising the temperature on competition. Surprisingly, strand displacement is much better at the higher temperature. It is all the more striking because of the usual poor competition seen when both hybrid and competitor DNA are circular. A possible explanation for these observations is that reagent complexes are more unstable in 2× SSC or rec A buffer solution at 44°C, but this is unlikely to be the complete answer based on examination of related data (cf. sample 1 of Table 2 of Example 7); a better explanation is that 44°C is closer to the optimal hybridization temperature in the absence of recombination protein.

Since the previous experiment suggests that strand displacement at 44°C is different from that at 37°C, a comparison was separately conducted to strand displacement at 40°C and 37°C.

The results suggest that the enhancement in strand displacement due to the use of rec A buffer solution and rec A protein is greatest at or near 40°C. This may be a very useful attribute of this diagnostic system when coupled to an appropriate non-isotopic detection method.

Example 10

Another means for altering the effect of rec A protein on nucleic acid strand displacement reactions is to perturb the degree of base complementarity between probe polynucleotides and labeled polynucleotides in reagent complexes. This approach was studied in the presence of *E. coli* rec A protein by comparing the extent of strand displacement from two reagent complexes; each contained 27 nucleotide long labeled oligonucleotides, but they differed in that one 27-mer contained a single base mismatch (see Table A preceding Example 1, above). Each reaction mixture employed 12 fmoles of linearized reagent complex prepared as in Example 5 and 12 fmoles of circular competitor DNA. Rec A protein was present in either 40-fold or 5-fold weight excess over reagent complex. Polyethylene glycol (PEG), MW 6000, was added to one sample. All reaction mixtures were incubated at 37°C as detailed in Example 5.

TABLE 5

| Samples | Percent unbound 27-mer | |
| --- | --- | --- |
| | Perfect match 27-mer | Single mismatched 27-mer |
| 1. Rec A buffer solution, no competitor DNA* | 44 | 37 |
| 2. Rec A buffer solution | 50 | 67 |
| 3. Rec A buffer solution, 40× weight excess of rec A protein | 100 | 100 |
| 4. Rec A buffer solution, 5× weight excess of rec A protein, PEG (MW 6000) | 92 | 100 |

*All other reaction mixtures contained competitor DNA.

23

**0 164 876**

The analyzed samples (Table 5) indicate rec A protein produces greater levels of strand displacement when present despite the existence of significant background due to contaminating free labeled polynucleotide. The enhancement seen with *E. coli* rec A protein still occurs using reagent complex carrying a single base pair mismatch between the labeled and probe polynucleotides. This observation suggests that there need not be perfect homology within the reagent complex in order to obtain beneficial effect from the presence and activity of the recombination protein.

**Claims**

1. A method for determining the presence of a predetermined target nucleotide sequence in the nucleic acid of a biological sample which comprises the steps:

(a) providing a reagent complex of (i) a probe polynucleotide which is capable of base pair binding via hydrogen bonds of purine/pyrimidine bases to the target nucleotide sequence, and (ii) a labeled polynucleotide which is bound by base pair binding via hydrogen bonds or purine/pyrimidine base pairs to the probe polynucleotide in a region of the probe polynucleotide at least partially coextensive with the region in which the probe polynucleotide is capable of binding to the target nucleotide sequence;

(b) contacting the reagent complex with a sample, in the presence of (1) an effective amount of a recombination protein having hybridization-enhancing activity and (2) cofactors necessary for its activity, under conditions in which the target nucleotide sequence, if present, binds to the probe polynucleotide and displaces labeled polynucleotide from the complex; and

(c) determining the presence of labeled polynucleotide displaced from the reagent complex.

2. The method of claim 1 wherein the probe polynucleotide contains a target binding region capable of base pair binding to the target nucleotide sequence and a labeled polynucleotide binding region bound to bases of the labeled polynucleotide in the complex, and wherein the labeled polynucleotide binding region is contained within the target binding region.

3. The method of claim 1 or 2 wherein the labeled polynucleotide binding region is about 20 to about 500 nucleotides in length.

4. The method of any previous claim wherein the portion of the target binding region that is not part of the labeled polynucleotide region is at least about 100 nucleotides in length.

5. The method of any previous claim wherein the recombination protein is of bacterial origin.

6. The method of claim 5 wherein the recombination protein is rec A protein from the enteric bacterium *E. coli* and the cofactors present comprise $Mg^{++}$ and ATP.

7. The method of any previous claim wherein the probe polynucleotide in the reagent complex is immobilized to a solid support.

8. The method of any previous claim wherein the determining step (c) comprises:

(c1) separating a first phase containing immobilized probe polynucleotide from a second phase comprising displaced labeled polynucleotide; and

(c2) detecting the presence of labeled polynucleotide in the second phase.

9. The method of any of claims 1—6 wherein the reagent complex is free in solution during the contacting step (b).

10. The method of claim 9 wherein the determining step (c) comprises:

(c1) separating the reagent complex remaining from a portion of the reaction solution after the contacting step (b), and

(c2) detecting the presence of any displaced labeled polynucleotide in a solution phase after separation.

11. The method of any previous claim wherein the probe polynucleotide is DNA.

12. A method for determining the presence of a predetermined target nucleotide sequence in the nucleic acid of a biological sample which comprises the steps:

(a) providing a reagent complex of (i) a probe polynucleotide which is capable of base pair binding via hydrogen bonds of purine/pyrimidine bases to the target nucleotide sequence, and (ii) a labeled polynucleotide which is bound by base pair binding via hydrogen bonds of purine/pyrimidine base pairs to the probe polynucleotide in a region of the probe polynucleotide at least partially coextensive with the region in which the probe polynucleotide is capable of binding to the target nucleotide sequence;

(b) contacting the reagent complex with a sample in the presence of (1) an effective amount of a recombination protein having hybridization-enhancing activity and (2) cofactors necessary for its activity, under conditions in which the target nucleotide sequence, if present, binds to the probe polynucleotide and displaces labeled polynucleotide from the complex; and

(c) determining the presence of labeled polynucleotide remaining in the reagent complex.

13. A diagnostic kit for determining the presence of a predetermined target nucleotide sequence in the nucleic acid of a biological sample comprising:

(a) a reagent complex of:

(i) a probe polynucleotide which is capable of base pair binding via hydrogen bonds of purine/pyrimidine base pairs to the target nucleotide sequence, and

(ii) a labeled polynucleotide which is bound by base pair binding via hydrogen bonds of purine/pyrimidine base pairs to the probe polynucleotide in a region of the probe polynucleotide at least

24

partially coextensive with the region in which the probe polynucleotide is capable of base pair binding to the target nucleotide sequence;

the potential base pair binding between the target nucleotide sequence and the probe polynucleotide being capable of displacing the labeled polynucleotide from the reagent complex.

(b) a recombination protein having hybridization-enhancing activity; and

(c) cofactors necessary for the activity of the recombination protein.

14. The diagnostic kit of claim 13 wherein the recombination protein is of bacterial origin.

15. The diagnostic kit of claim 13 or 14 wherein the probe polynucleotide is DNA.

**Patentansprüche**

1. Verfahren zur Bestimmung der Gegenwart einer vorbestimmten Zielnucleotidsequenz in der Nucleinsäure einer biologischen Probe mit den Stufen, bei denen man

(a) einen Reagenzkomplex von (i) einem Prüfpolynucleotid, das zur Basenpaarbindung über Wasserstoffbindungen von Purin/Pyrimidinbasen an die Zielnucleotidsequenz in der Lage ist, und (ii) eines markierten Polynucleotids, das durch Basenpaarbindung über Wasserstoffbindungen von Purin/Pyrimidinbasenpaaren an das Prüfpolynucleotid in einem Bereich des Prüfpolynucleotids, das sich wenigstens teilweise über den Bereich erstreckt, in welchem das Prüfpolynucleotid sich an die Zielnucleotidsequenz binden kann, gebunden ist, vorsieht,

(b) den Reagenzkomplex mit einer Probe in Gegenwert (1) einer wirksamen Menge eines Rekombinationsproteins mit hybridisierungssteigernder Aktivität und (2) von für seine Aktivität erforderlichen Cofaktoren unter Bedingungen in Berührung bringt, bei denen die Zielnucleotidsequenz, wenn vorhanden, sich an das Prüfpolynucleotid bindet und markiertes Polynucleotid aus dem Komplex verdrängt, und

(c) das Vorhandensein von markiertem Polynucleotid, das aus dem Reagenzkomplex verdrängt wurde, bestimmt.

2. Verfahren nach Anspruch 1, bei dem das Prüfpolynucleotid einen Zielbindungsbereich enthält, der zu einer Basenpaarbindung an die Zielnucleotidsequenz in der Lage ist, und einen Bindungsbereich für markiertes Polynucleotid enthält, der an Basen des markierten Polynucleotids in dem Komplex gebunden ist, und bei dem der Bindungsbereich für markiertes Polynucleotid in dem Zielbindungsbereich enthalten ist.

3. Verfahren nach Anspruch 1 oder 2, bei dem der Bindungsbereich für markiertes Polynucleotid eine Länge von etwa 20 bis etwa 500 Nucleotiden hat.

4. Verfahren nach einem der vorausgehenden Ansprüche, bei dem der Abschnitt des Zielbindungsbereiches, der nicht Teil des Bindungsbereichs für markiertes Polynucleotid ist, eine Länge von wenigstens 100 Nucleotiden hat.

5. Verfahren nach einem der vorausgehenden Ansprüche, bei dem das Rekombinationsprotein bakteriellen Ursprungs ist.

6. Verfahren nach Anspruch 5, bei dem das Rekombinationsprotein rec. A-Protein aus dem Darmbakterium E. coli ist und die vorhandenen Cofaktoren $Mg^{++}$ und ATP aufweisen.

7. Verfahren nach einem der vorausgehenden Ansprüche, bei dem das Prüfpolynucleotid in dem Reagenzkomplex durch Bindung an einen festen Träger immobilisiert ist.

8. Verfahren nach einem der vorausgehenden Ansprüche, bei dem die Bestimmungsstufe (c) darin besteht, daß man

(c1) eine erste Phase, die immobilisiertes Prüfpolynucleotid enthält, von einer zweiten Phase, die verdrängtes markiertes Polynucleotid aufweist, trennt und

(c2) das Vorhandensein von markiertem Polynucleotid in der zweiten Phase ermittelt.

9. Verfahren nach einem der Ansprüche 1 bis 6, bei dem der Reagenzkomplex während der Behandlungsstufe (b) frei in Lösung ist.

10. Verfahren nach Anspruch 9, bei dem die Bestimmungsstufe (c) darin besteht, daß man

(c1) den Reagenzkomplex, der nach der Behandlungsstufe (b) in einem Teil der Reaktionslösung bleibt, abtrennt und

(c2) das Vorhandensein von verdrängtem markiertem Polynucleotid in einer Lösungsphase nach der Abtrennung ermittelt.

11. Verfahren nach einem der vorausgehenden Ansprüche, bei dem das Prüfpolynucleotid DNA ist.

12. Verfahren zur Bestimmung des Vorhandenseins einer vorbestimmten Zielnucleotidsequenz in der Nucleinsäure einer biologischen Probe mit den Stufen, bei denen man

(a) einen Reagenzkomplex (i) eines Prüfpolynucleotids, das zur Basenpaarbindung über Wasserstoffbindungen von Purin/Pyrimidinbasen an die Zielnucleotidsequenz in der Lage ist, und (ii) eines markierten Polynucleotids, das durch Basenpaarbindung über Wasserstoffbindungen von Purin/Pyrimidinbasenpaaren an das Prüfpolynucleotid in einem Bereich des Prüfpolynucleotids, der sich wenigstens teilweise über den Bereich erstreckt, in welchem das Prüfpolynucleotid zur Bindung an die Zielnucleotidsequenz in der Lage ist, gebunden ist, vorsieht,

(b) den Reagenzkomplex mit einer Probe in Gegenwart (1) einer wirksamen Menge eines Rekombinationsproteins mit hybridisierungssteigernder Aktivität und (2) für seine Aktivität erforderlicher Cofaktoren unter Bedingungen, bei denen die Zielnucleotidsequenz, wenn vorhanden, sich an das Prüfpolynucleotid bindet und markiertes Polynucleotid aus dem Komplex verdrängt, in Berührung bringt und

(c) das Vorhandensein von in dem Reagenzkomplex verbleibendem markiertem Polynucleotid bestimmt.

13. Diagnostisches Mittel zur Bestimmung des Vorhandenseins einer vorbestimmten Zielnucleotidsequenz in der Nucleinsäure einer biologischen Probe mit:

(a) einem Reagenzkomplex

(i) eines Prüfpolynucleotids, das zur Basenpaarbindung über Wasserstoffbindungen von Purin/Pyrimidinbasenpaaren an die Zielnucleotidsequenz in der Lage ist, und

(ii) eines markierten Polynucleotids, das durch Basenpaarbindung über Wasserstoffbindungen von Purin/Pyrimidinbasenpaaren an das Zielpolynucleotid in einem Bereich des Zielpolynucleotids, der sich wenigstens teilweise über den Bereich erstreckt, in welchem das Zielpolynucleotid zur Basenpaarbindung an die Zielnucleotidsequenz in der Lage ist, gebunden ist.

wobei die potentielle Basenpaarbindung zwischen der Zielnucleotidsequenz und dem Prüfpolynucleotid in der Lage ist, das markierte Polynucleotid aus dem Reagenzkomplex zu verdrängen,

(b) einem Rekombinationsprotein mit hybridisierungssteigernder Aktivität und

(c) Cofaktoren, die für die Aktivität des Rekombinationsproteins erforderlich sind.

14. Diagnostisches Mittel nach Anspruch 13, bei dem das Rekombinationsprotein bakteriellen Ursprungs ist.

15. Diagnostisches Mittel nach Anspruch 13 oder 14, bei dem das Prüfpolynucleotid DNA ist.


**Revendications**

1. Un procédé pour la détermination de la présence d'une séquence cible prédéterminée de nucléotides dans l'acide nucléique d'un échantillon biologique qui comprend les stades consistant à:

(a) se pourvoir d'un complexe réactif de (i) un polynucléotide sonde capable de liaison de paires de bases par l'intermédiaire de liaisons hydrogène de bases puriques/pyrimidiques avec la séquence cible de nucléotides et (ii) un polynucléotide marqué qui est lié par liaison de paires de bases par l'intermédiaire de liaisone hydrogène de paires de bases puriques/pyrimidiques au polynucléotide sonde dans une région du polynucléotide sonde s'étendant au moins partiellement dans la région où le polynucléotide sonde est capable de liaison avec la séquence cible de nucléotides;

(b) mettre le complexe réactif au contact d'un échantillon en présence de (1) une quantité efficace d'une protéine de recombinaison ayant une activité d'accroissement de l'hybridation et (2) des cofacteurs nécessaires à son activité, dans des conditions telles que la séquence cible de nucléotides, lorsqu'elle est présente, se lie au polynucléotide sonde et déplace le polynucléotide marqué du complexe; et

(c) déterminer la présence du polynucléotide marqué déplacé du complexe réactif.

2. Le procédé de la revendication 1 dans lequel le polynucléotide sonde contient une région de liaison de la cible capable de liaison de paires de basse avec la séquence cible de nucléotides et une région de liaison de nucléotide marqué liée aux bases du polynucléotide marqué dans le complexe et dans lequel la région de liaison du polynucléotide marqué est contenue dans la région de liaison de la cible.

3. Le procédé de la revendication 1 ou 2 dans lequel la région de liaison du polynucléotide marqué a une longueur d'environ 20 à environ 500 nucléotides.

4. Le procédé de l'une quelconque des revendications précédentes dans lequel la portion de la région de liaison de la cible, qui ne fait pas partie de la région de liaison du polynucléotide marqué, a une longueur d'au moins environ 100 nucléotides.

5. Le procédé de l'une quelconque des revendications précédentes dans lequel la protéine de recombinaison est d'origine bactérienne.

6. Le procédé de la revendication 5 dans lequel la protéine de recombinaison est la protéine roc à de l'entérobactérie *E. coli*, et les cofacteurs présents comprennent $Mg^{++}$ et l'ATP.

7. Le procédé de l'une quelconque des revendications précédentes dans lequel le polynucléotide sonde, dans le complexe réactif, est immobilisé à une support solide.

8. Le procédé de l'une quelconque des revendications précédentes, dans lequel le stade de détermination (c) comprend:

(c1) la séparation d'une première phase contenant le polynucléotide sonde immobilisé d'avec une seconde phase comprenant le polynucléotide marqué déplacé; et

(c2) la détection de la présence du polynucléotide marqué dans la seconde phase.

9. Le procédé de l'une quelconque des revendications 1 à 6 dans lequel le complexe réactif est libre en solution pendant le stade de contact (b).

10. Le procédé de la revendication 9 dans lequel le stade de détermination (c) comprend:

(c1) la séparation du complexe réactif restant d'avec une portion de la solution réactionnelle après le stade de contact (b), et

(c2) la détection de la présence de tout nucléotide marqué déplacé dans une phase de solution après le séparation.

26

**0 164 876**

11. Le procédé de l'une quelconque des revendications précédentes dans lequel le polynucléotide sonde est un ADN.

12. Un procédé pour la détermination de la présence d'une séquence cible prédéterminée de nucléotides dans l'acide nucléique d'un échantillon biologique qui comprend les stades consistant à:

(a) se pourvoir d'un complexe réactif de (i) un polynucléotide sonde capable de liaison de paires de bases par l'intermédiaire liaisons hydrogène de bases puriques/pyrimidiques avec la séquence cible de nucléotides et (ii) un polynucléotide marqué qui est lié par liaison de paires de bases par l'intermédiaire de liaisons hydrogène de paires de bases puriques/pyrimidiques au polynucléotide sonde dans une région du polynucléotide sonde s'étendant au moins partiellement dans la région où le polynucléotide sonde est capable de liaison avec la séquence cible de nucléotides;

(b) mettre le complexe réactif au contact d'un échantillon en présence de (1) une quantité efficace d'une protéine de recombinaison ayant une activité d'accroissement de l'hybridation et (2) des cofacteurs nécessaires à son activité, dans des conditions telles que la séquence cible de nucléotides, lorsqu'elle est présente, se lie au polynucléotide sonde et déplace le polynucléotide marqué du complexe; et

(c) déterminer la présence du polynucléotide marqué demeurant dans le complexe réactif.

13. Une trousse diagnostique pour la détermination de la présence d'une séquence cible prédéterminée de nucléotides dans l'acide nucléique d'un échantillon biologique, comprenant:

(a) un complexe réactif de:

(i) un polynucléotide sonde qui est capable de liaison de paires de bases par l'intermédiaire de liaisons hydrogène de paires de bases puriques/pyrimidiques avec la séquence cible de nucléotides, et

(ii) un polynucléotide marqué qui est lié par liaison de paires de bases par l'intermédiaire de liaisons hydrogène de paires de bases puriques/pyrimidiques au polynucléotide sonde dans une région du polynucléotide sonde s'étendant au moins partiellement dans la région où le polynucléotide sonde est capable de liaison de paires de bases avec la séquence cible de nucléotides;

la liaison de paires de bases potientielle entre la séquence cible de nucléotides et le polynucléotide sonde étant capable de déplacer le polynucléotide marqué du complexe réactif;

(b) une protéine de recombinaison ayant une activité d'accroissement de l'hybridation; et

(c) des cofacteurs nécessaires à l'activité de la protéine de recombinaison.

14. La trousse diagnostique de la revendication 13 dans laquelle la protéine de recombinaison est d'origine bactérienne.

15. La trousse diagnostique de la revendication 13 ou 14 dans laquelle le polynucléotide sonde est un ADN.

27

FIG. IA

FIG. IB

FIG. IC

FIG.1D

FIG.1E

FIG.1F

FIG.1G

FIG. 2

FIG.4

FIG.5

FIG.6

4

FIG.3A

FIG.3B

FIG.3C

FIG.3D